(19) Europäisches Patentamt
European Patent Office
Office européen des brevets

(11) Veröffentlichungsnummer: **0 248 315**
**A2**

(12) **EUROPÄISCHE PATENTANMELDUNG**

(21) Anmeldenummer: **87107586.7**

(22) Anmeldetag: **25.05.87**

(51) Int. Cl.4: **C07D 231/40** , C07D 401/04 , C07D 403/04 , A01N 43/56 , A01N 43/40 , A01N 43/36

(30) Priorität: **04.06.86 DE 3618717**

(43) Veröffentlichungstag der Anmeldung: **09.12.87 Patentblatt 87/50**

(84) Benannte Vertragsstaaten: **AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Stetter,Jörg, Dr.**
**Gellertweg 4**
**D-5600 Wuppertal 1(DE)**
Erfinder: **Schallner, Otto, Dr.**
**Noldeweg 22**
**D-4019 Monheim(DE)**
Erfinder: **Gehring, Reinhold, Dr.**
**Dasnoeckel 49**
**D-5600 Wuppertal 11(DE)**
Erfinder: **Santel, Hans-Joachim, Dr.**
**Gruenstrasse 9a**
**D-5090 Leverkusen(DE)**
Erfinder: **Schmidt, Robert R., Dr.**
**Im Waldwinkel 110**
**D-5060 Bergisch-Gladbach 2(DE)**
Erfinder: **Lürssen, Klaus, Dr.**
**August-Kierspel-Strasse 151**
**D-5060 Bergisch-Gladbach 2(DE)**

(54) **5-Acylamino-pyrazol-Derivate.**

(57) Die Erfindung betrifft 5-Acylamino-pyrazol-Derivate der allgemeinen Formel (I)

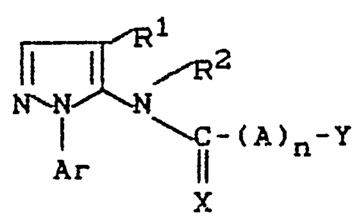

(I)

in welcher
$R^1$ für Wasserstoff, Halogen oder Nitro steht,
$R^2$ für Wasserstoff, Alkyl, Alkenyl, Alkinyl oder gegebenenfalls substituiertes Cycloalkyl steht,
Ar für jeweils gegebenenfalls substituiertes Phenyl oder Pyridyl steht,
X für Sauerstoff oder Schwefel steht,
A für eine geradkettige oder verzweigte, gegebenenfalls substituierte Alkylenbrücke steht,
n für die Zahlen 0 oder 1 steht und

EP 0 248 315 A2

Y für Cyano oder die Gruppierung

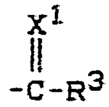

steht, wobei
$X^1$ und $R^3$ die in der Beschreibung angegebene Bedeutung haben,
mehrere Verfahren zu ihrer Herstellung und ihre Verwendung als Herbizide und Wachstumsregulatoren sowie neue Zwischenprodukte.

## 5-Acylamino-pyrazol-Derivate

Die Erfindung betrifft neue 5-Acylamino-pyrazol-Derivate, mehrere Verfahren zu ihrer Herstellung sowie ihre Verwendung als Herbizide und Wachstumsregulatoren.

Es ist bereits bekannt, daß bestimmte 5-Acylamido-1-aryl-pyrazole, wie beispielsweise das 4-Cyano-5-propionamido-1-(2,4,6-trichlorphenyl)-pyrazol herbizide Eigenschaften besitzen (vgl. z.B. DE-OS 3 226 513).

Die herbizide Wirksamkeit dieser vorbekannten Verbindungen gegenüber Unkräutern istjedoch ebenso wie ihre Verträglichkeit gegenüber wichtigen Kulturpflanzen nicht immer in allen Anwendungsbereichen völlig zufriedenstellend.

Es wurden neue 5-Acylamino-pyrazol-Derivate der allgemeinen Formel (I)

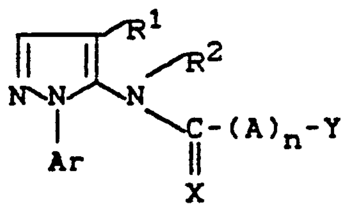

(I)

in welcher
$R^1$ für Wasserstoff, Halogen oder Nitro steht,
$R^2$ für Wasserstoff, Alkyl, Alkenyl, Alkinyl oder gegebenenfalls substituiertes Cycloalkyl steht,
Ar für jeweils gegebenenfalls substituiertes Phenyl oder Pyridyl steht,
X für Sauerstoff oder Schwefel steht,
A für eine geradkettige oder verzweigte, gegebenenfalls substituierte Alkylenbrücke steht,
n für die Zahlen 0 oder 1 steht und
Y für Cyano oder die Gruppierung

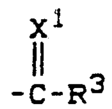

steht, wobei
$X^1$ für Sauerstoff oder Schwefel steht und
$R^3$ für Hydroxy, Alkoxy, Alkenyloxy, Alkinyloxy, Alkylthio, Amino, Alkylamino, Dialkylamino, Alkenylamino, Dialkenylamino, Alkyl-alkenylamino oder einen Rest -OM steht, wobei
M für ein Äquivalent des Kations einer anorganischen oder organischen Base steht,
gefunden.

Weiterhin wurde gefunden, daß man die neuen 5-Acyl-amino-pyrazol-Derivate der Formel (I)

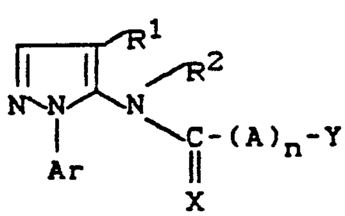

(I)

in welcher
$R^1$ für Wasserstoff, Halogen oder Nitro steht,
$R^2$ für Wasserstoff, Alkyl, Alkenyl, Alkinyl oder gegebenenfalls substituiertes Cycloalkyl steht,
Ar für jeweils gegebenenfalls substituiertes Phenyl oder Pyridyl steht,
X für Sauerstoff oder Schwefel steht,
A für eine geradkettige oder verzweigte, gegebenenfalls substituierte Alkylenbrücke steht,
n für die Zahlen 0 oder 1 steht und
Y für Cyano oder die Gruppierung

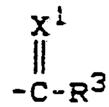

steht, wobei
$X^1$ für Sauerstoff oder Schwefel steht und
$R^3$ für Hydroxy, Alkoxy, Alkenyloxy, Alkinyloxy, Alkylthio, Amino, Alkylamino, Dialkylamino, Alkenylamino, Dialkenylamino, Alkyl-alkenylamino oder einen Rest OM steht, wobei
M für ein Äquivalent des Kations einer anorganischen oder organischen Base steht,
mit Hilfe der im folgenden beschriebenen Verfahren erhält:

a) Man erhält 5-Acylamino-pyrazol-Derivate der Formel (Ia)

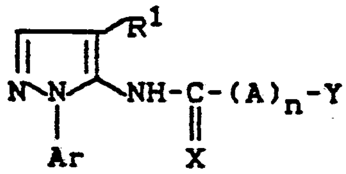 (Ia)

in welcher
$R^1$, X, Y, A, Ar und der Index n die oben angegebene Bedeutung haben,
wenn man 5-Amino-1-aryl-pyrazole der Formel (II)

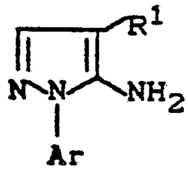 (II)

in welcher
$R^1$ und Ar die oben angegebene Bedeutung haben,
mit Acylierungsmitteln der Formel (III)

$E^1$ - C -$(A)_n$ -Y
‖
X
(III)

in welcher
X, Y, A und der Index n die oben angegebene Bedeutung haben und
$E^1$ für eine elektronenanziehende Abgangsgruppe steht,
gegebenenfalls in Gegenwart eines Verdünnungsmittels, gegebenenfalls in Gegenwart eines Säurebindemittels sowie gegebenenfalls in Gegenwart eines Katalysators umsetzt;

b) Man erhält 5-Acylamino-pyrazol-Derivate der Formel (Ib)

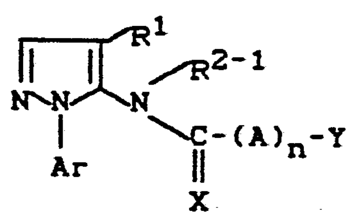 (Ib)

in welcher
$R^1$, X, Y, A, der Index n und Ar die oben angegebene Bedeutung haben und
$R^{2-1}$ für Alkyl, Alkenyl, Alkinyl oder gegebenenfalls substituiertes Cycloalkyl steht,
wenn man die nach Verfahren (a) erhältlichen 5-Acylamino-pyrazol-Derivate der Formel (Ia)

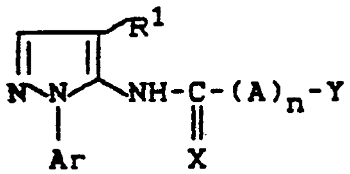

(Ia)

in welcher
$R^1$, X, Y, A, Ar und der Index n die oben angegebene Bedeutung haben,
mit Alkylierungsmitteln der Formel (IV)
$E^2$ -$R^{2-1}$ (IV)
in welcher
$R^{2-1}$ die oben angegebene Bedeutung hat und
$E^2$ für Halogen, für gegebenenfalls substituiertes Alkoxysulfonyloxy oder für gegebenenfalls substituiertes Arylsulfonyloxy steht,
gegebenenfalls in Gegenwart eines Verdünnungsmittels und gegebenenfalls in Gegenwart eines Säurebindemittels sowie gegebenenfalls in Gegenwart eines Katalysators umsetzt;

c) Man erhält 5-Acylamino-pyrazol-Derivate der Formel (Ic)

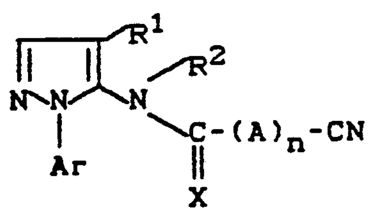

(Ic)

in welcher
$R^1$, $R^2$, Ar, X, A und der Index n die oben angegebene Bedeutung haben,
wenn man 5-Acylamido-1-aryl-pyrazole der Formel (V)

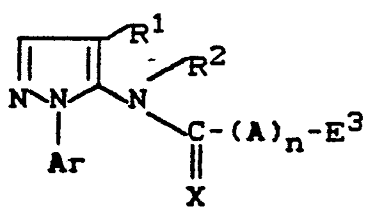

(V)

in welcher
$R^1$, $R^2$, Ar, X, A und der Index n die oben angegebene Bedeutung haben und
$E^3$ für Halogen steht,
mit Cyan-Derivaten der Formel (VI)
W-CN (VI)
in welcher
W für Wasserstoff, ein Äquivalent eines Erdalkali-oder Alkalimetallkations oder für ein Äquivalent des Kations einer organischen Base steht,
gegebenenfalls in Gegenwart eines Verdünnungsmittels und gegebenenfalls in Gegenwart eines Säurebindemittels sowie gegebenenfalls in Gegenwart eines Katalysators umsetzt;

d) Man erhält 5-Acylamino-pyrazol-Derivate der Formel (Id)

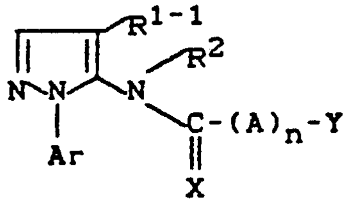

(Id)

in welcher
$R^2$, X, A, Y, Ar und der Index n die oben angegebene Bedeutung haben und
$R^{1-1}$ für Halogen oder Nitro steht,
wenn man die mit Hilfe der Verfahren (a), (b), (c), (e) oder (f) erhältlichen 5-Acylamino-pyrazol-Derivate der Formel (Ie),

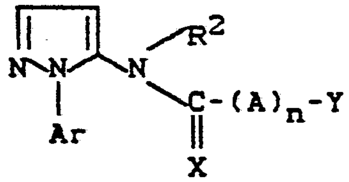

(Ie)

in welcher
$R^2$, X, Y, A, Ar und der Index n die oben angegebene Bedeutung haben,
mit Halogenierungs-oder Nitrierungsmitteln der Formel (VII)
$R^{1-1}$ -$E^4$ (VII)
in welcher
$R^{1-1}$ die oben angegebene Bedeutung hat und
$E^4$ für eine elektronenanziehende Abgangsgruppe steht,
gegebenenfalls in Gegenwart eines Verdünnungsmittels und gegebenenfalls in Gegenwart eines Katalysators oder Reaktionshilfsmittels umsetzt;

e) Man erhält 5-Acylamino-Pyrazol-Derivate der Formel (If)

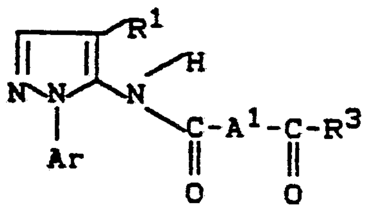

(If)

in welcher
$R^1$, $R^3$ und Ar die oben angegebene Bedeutung haben und
$A^1$ für geradkettiges oder verzweigtes Alkylen mit 1 bis 6 Kohlenstoffatomen steht,
wenn man 5-Imido-pyrazole der Formel (VIII)

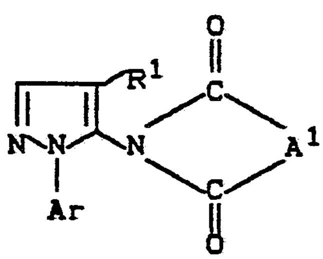

(VIII)

in welcher
$R^1$, Ar, und $A^1$ die oben angegebene Bedeutung haben,
mit nucleophilen Verbindungen der Formel (IX)
$R^3$ -H (IX)
in welcher
$R^3$ die oben angegebene Bedeutung hat,
gegebenenfalls in Gegenwart eines Verdünnungsmittels und gegebenenfalls in Gegenwart eines Reaktionshilfsmittels umsetzt;

f) Man erhält 5-Acylamino-pyrazol-Derivate der Formel (Ig)

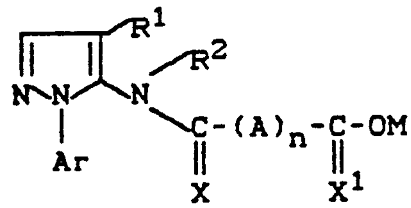

(Ig)

in welcher
$R^1$, $R^2$, Ar, A, X, $X^1$, M und der Index n die oben angegebene Bedeutung haben
wenn man die nach den Verfahren (a), (b), (d) oder (e) erhältlichen 5-Acylamino-pyrazol-Derivate der Formel (Ih)

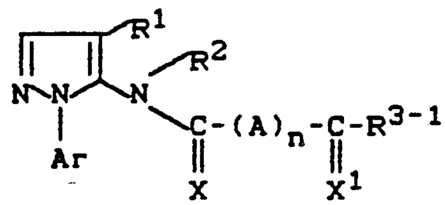

(Ih)

in welcher
$R^1$, $R^2$, Ar, X, $X^1$, A und der Index n die oben angegebene Bedeutung haben und
$R^{3-1}$ für Hydroxy oder Alkoxy steht,
mit den Hydroxiden oder Carbonaten von Alkali-oder Erdalkalimetallen neutralisiert oder verseift, bzw. mit organischen Basen neutralisiert.

Schließlich wurde gefunden, daß die neuen 5-Acylamino-pyrazol-Derivate der allgemeinen Formel (I) herbizide, insbesondere auch selektiv-herbizide und wachstumsregulierende Eigenschaften besitzen. Überraschenderweise zeigen die erfindungsgemäßen 5-Acylamino-pyrazol-Derivate der allgemeinen Formel (I) eine erheblich bessere allgemein-herbizide Wirksamkeit gegen schwer bekämpfbare Problemunkräuter und gleichzeitig eine deutlich verbesserte Verträglichkeit gegenüber wichtigen Kulturpflanzen im Vergleich zu den aus dem Stand der Technik bekannten 5-Acylamido-1-aryl-pyrazolen, wie beispielsweise dem 4-Cyano-5-propionamido-1-(2,4,6-trichlorphenyl)-pyrazol, welche chemisch und wirkungsmäßig naheliegende Verbindungen sind.

Die erfindungsgemäßen 5-Acylamino-pyrazol-Derivate sind durch die Formel (I) allgemein definiert. Bevorzugt sind Verbindungen der Formel (I), bei welchen
$R^1$ für Wasserstoff, Nitro, Fluor, Chlor, Brom oder Iod steht;
$R^2$ für Wasserstoff, für geradkettiges oder verzweigtes Alkyl mit 1 bis 12 Kohlenstoffatomen, für jeweils geradkettiges oder verzweigtes Alkenyl und Alkinyl mit jeweils 3 bis 8 Kohlenstoffatomen sowie für gegebenenfalls durch Alkyl mit 1 bis 4 Kohlenstoffatomen substituiertes Cycloalkyl mit 3 bis 8 Kohlenstoffatomen steht;
Ar für jeweils gegebenenfalls einfach oder mehrfach gleich oder verschieden substituiertes Phenyl, 2-Pyridyl, 3-Pyridyl oder 4-Pyridyl steht, wobei als Substituenten jeweils infrage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest - $S(O)_m$ -$R^4$,
wobei
$R^4$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und m für eine Zahl 0, 1 oder 2 steht; sowie
X für Sauerstoff oder Schwefel steht;
A für eine geradkettige oder verzweigte, gegebenenfalls einfach oder mehrfach, gleich oder verschieden durch Halogen oder Phenyl, das gegebenenfalls durch Halogen oder Alkyl mit 1 bis 4 Kohlenstoffatomen substiuiert ist, substituierte Alkylenbrücke mit 1 bis 6 Kohlenstoffatomen steht;
n für eine Zahl 0 oder 1 steht und
Y für Cyano oder die Gruppierung

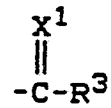

steht,
wobei
$X^1$ für Sauerstoff oder Schwefel steht und
$R^3$ für Hydroxy, jeweils geradkettiges oder verzweigtes Alkoxy und Alkylthio mit jeweils 1 bis 6 Kohlenstoffatomen, jeweils geradkettiges oder verzweigtes Alkenyloxy und Alkinyloxy mit jeweils 3 bis 8 Kohlenstoffatomen, Amino, jeweils geradkettiges oder verzweigtes Alkylamino und Dialkylamino mit jeweils 1 bis 6 Kohlenstoffatomen in den Alkylteilen, jeweils geradkettiges oder verzweigtes Alkenylamino und Dialkenylamino mit jeweils 3 bis 8 Kohlenstoffatomen in den Alkenylteilen, geradkettiges oder verzweigtes Alkylalkenylamino mit 1 bis 6 Kohlenstoffatomen im Alkylteil und 3 bis 8 Kohlenstoffatomen im Alkenylteil steht, oder für einen Rest -OM steht,
wobei
M für ein Äquivalent eines Erdalkali-oder Alkalimetallkations oder für ein gegebenenfalls ein-bis vierfach, gleich oder verschieden durch geradkettiges oder verzweigtes Alkyl mit 1 bis 12 Kohlenstoffatomen oder Benzyl substituiertes Ammonium steht.

Besonders bevorzugt sind 5-Acylamino-pyrazol-Derivate der Formel (I), bei welchen
$R^1$ für Wasserstoff, Nitro, Chlor oder Brom steht;
$R^2$ für Wasserstoff, für geradkettiges oder verzweigtes Alkyl mit 1 bis 8 Kohlenstoffatomen, für jeweils geradkettiges oder verzweigtes Alkenyl und Alkinyl mit jeweils 3 bis 6 Kohlenstoffatomen sowie für gegebenenfalls durch Methyl, Ethyl oder Isopropyl substituiertes Cycloalkyl mit 3 bis 6 Kohlenstoffatomen steht;
Ar für gegebenenfalls ein-bis fünffach, gleich oder verschieden substituiertes Phenyl oder für jeweils gegebenenfalls ein-bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$,
wobei
$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und
m für eine Zahl 0, 1 oder 2 steht sowie
X für Sauerstoff oder Schwefel steht;
A für ein Brückenglied der Formel $-CH_2-$; $-CH_2-CH_2-$;

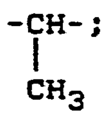 ; $-CCl_2-CH_2-$; $-CHCl-CH_2-$, 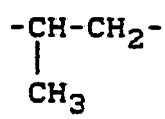 ;

$-CH_2-CH_2-CH_2-$;

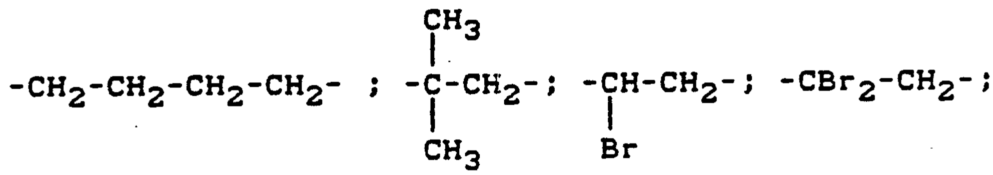

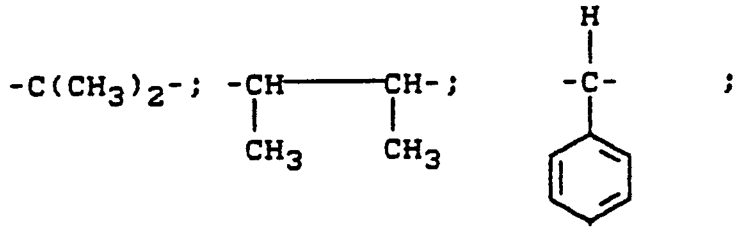

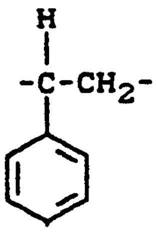 steht;

n für eine Zahl 0 oder 1 steht und
Y für Cyano oder die Gruppierung

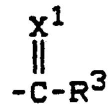

steht,
wobei
$X^1$ für Sauerstoff steht und
$R^3$ für Hydroxy, Alkoxy mit 1 bis 3 Kohlenstoffatomen, Alkenyloxy und Alkinyloxy mit jeweils 3 bis 6 Kohlenstoffatomen, Amino, Alkylamino und Dialkylamino mit jeweils 1 bis 3 Kohlenstoffatomen in jedem Alkylteil, Alkenylamino und Dialkenylamino mit jeweils 3 bis 6 Kohlenstoffatomen in jedem Alkenylteil, Alkylalkenylamino mit 1 bis 3 Kohlenstoffatomen im Alkylteil und 3 bis 6 Kohlenstoffatomen im Alkenylteil steht, oder für einen Rest -OM steht, wobei
M für ein Äquivalent eines Natrium-, Kalium-, Calcium-, Magnesium-oder Barium-Kations oder für ein Isopropylammonium-, Dimethylbenzylammonium-, Triethylammonium-oder Tributylammonium-Ion steht.

Ganz besonders bevorzugt sind 5-Acylamino-pyrazol-Derivate der Formel (I), bei welchen
$R^1$ für Wasserstoff oder Nitro steht;
$R^2$ für Wasserstoff, Methyl, Ethyl, Allyl, Propargyl oder Cyclohexyl steht;
Ar für gegebenenfalls ein-bis fünffach, gleich oder verschieden substituiertes Phenyl oder für jeweils gegebenenfalls ein-bis vierfach, gleich oder verschieden substituiertes 2-Pyridyl, 3-Pyridyl oder 4-Pyridyl steht, wobei als Substituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Difluorchlormethyl, Difluorchorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl Pentachlorethyl, Trifluormethoxy, Trichlormethoxy, Dichlorfluormethoxy, Difluorchlormethoxy, Chlormethoxy, Dichlormethoxy, Difluor methoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$,
wobei
$R^4$ für Amino, Methylamino, Ethylamino, Dimethylamino, Diethylamino, Fluordichlormethyl, Difluormethyl,

Tetrafluorethyl, Trifluorchlorethyl, Trifluormethyl, Methyl oder Ethyl steht und
m für eine Zahl 0, 1 oder 2 steht sowie
X für Sauerstoff oder Schwefel steht;
A für ein Brückenglied der Formel $-CH_2-$; $-CH_2-CH_2-$;

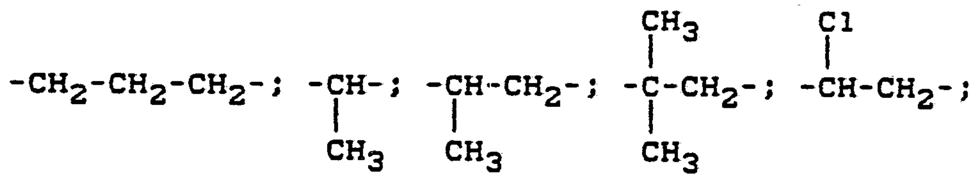

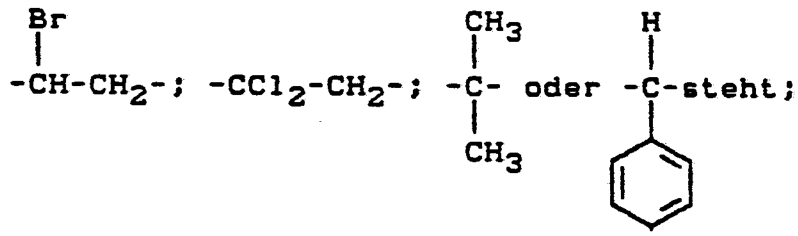
oder
steht;

n für eine Zahl 0 oder 1 steht und
Y für Cyano oder die Gruppierung

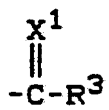

steht,
$X^1$ für Sauerstoff steht und
$R^3$ für Hydroxy, Methoxy, Ethoxy, n-und i-Propoxy Allyloxy, Propargyloxy, Amino, Methylamino, Dimethylamino, Allylamino, Diallylamino, Methylethylamino, Methyl-allylamino oder den Rest -OM steht,
wobei
M für ein Äquivalent eines Natrium-, Kalium-oder Calcium-Kations oder für ein Isopropylammonium-Ion steht.

Im einzelnen seien außer den bei den Herstellungsbeispielen genannten Verbindungen die folgenden 5-Acylaminopyrazol-Derivate der allgemeinen Formel (I) genannt:

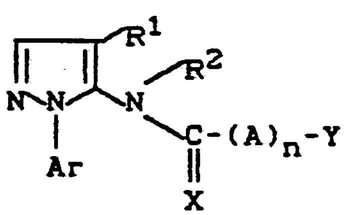

(I)

Tabelle 1

| $R^1$ | $R^2$ | X | n | A | Y | Ar |
|---|---|---|---|---|---|---|
| H | H | O | O | - | COOH | 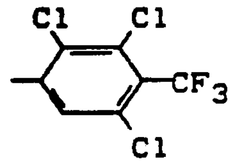 |
| H | H | O | O | - | COONa | 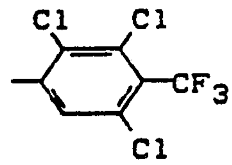 |
| H | H | O | O | - | COOK | 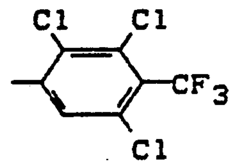 |
| H | H | O | O | - | $COONH_3C_3H_7$-i | 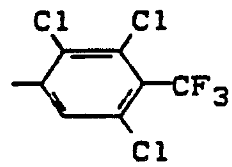 |
| $NO_2$ | H | O | O | - | COOH | 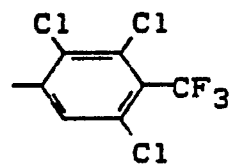 |
| $NO_2$ | H | O | O | - | COONa | 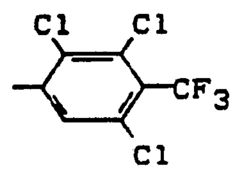 |
| $NO_2$ | H | O | O | - | COOK | 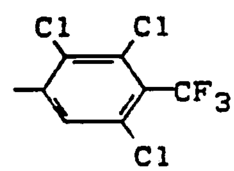 |
| $NO_2$ | H | O | O | - | $COONH_3C_3H_7$-i | 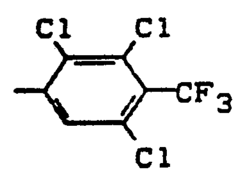 |

Tabelle 1 - Fortsetzung

| $R^1$ | $R^2$ | X | n | A | Y | Ar |
|---|---|---|---|---|---|---|
| $NO_2$ | H | O | O | - | COOH | 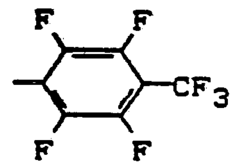 |
| $NO_2$ | H | O | O | - | $COOCH_3$ | 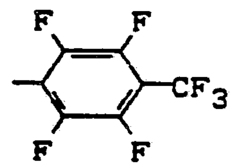 |
| $NO_2$ | H | O | O | - | $COOC_2H_5$ | 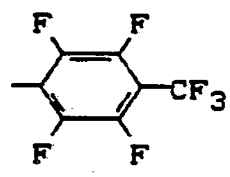 |
| $NO_2$ | H | O | O | - | $COOCH_3$ | 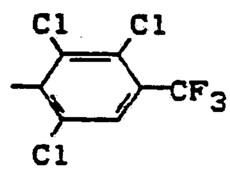 |
| $NO_2$ | H | O | O | - | $COOC_2H_5$ | 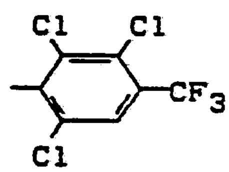 |
| $NO_2$ | H | O | O | - | $COOCH_3$ | 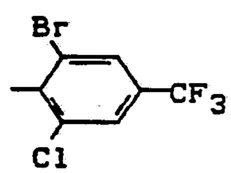 |
| $NO_2$ | H | O | O | - | $COOC_2H_5$ | 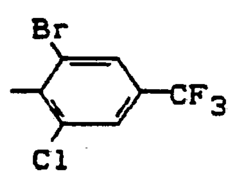 |

<u>Tabelle 1</u> - Fortsetzung

| $R^1$ | $R^2$ | X | n | A | Y | Ar |
|---|---|---|---|---|---|---|
| $NO_2$ | H | O | 0 | - | COOH | 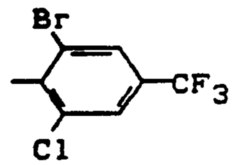 |
| $NO_2$ | H | O | 0 | - | COONa | 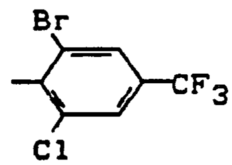 |
| $NO_2$ | H | O | 0 | - | $COOCH_3$ | 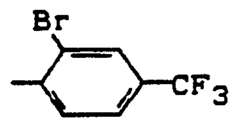 |
| $NO_2$ | H | O | 0 | - | COOH | 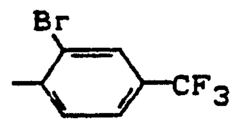 |
| $NO_2$ | H | O | 0 | - | $COOCH_3$ | 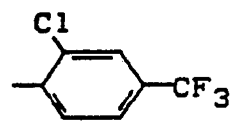 |
| $NO_2$ | H | O | 0 | - | $COOC_2H_5$ | 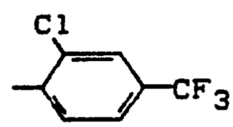 |
| $NO_2$ | H | O | 0 | - | COOH | 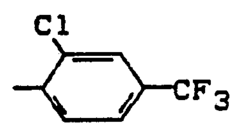 |
| $NO_2$ | H | O | 0 | - | $COOCH_3$ | 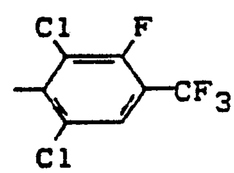 |

Tabelle 1 - Fortsetzung

| $R^1$ | $R^2$ | X | n | A | Y | Ar |
|---|---|---|---|---|---|---|
| $NO_2$ | H | O | 0 | - | $COOC_2H_5$ | 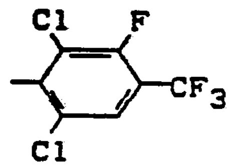 |
| $NO_2$ | H | O | 0 | - | COOH | 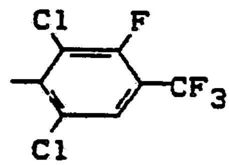 |
| $NO_2$ | H | O | 0 | - | $COOCH_3$ | 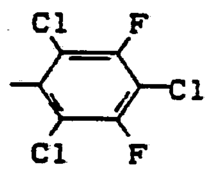 |
| $NO_2$ | H | O | 0 | - | COOH | 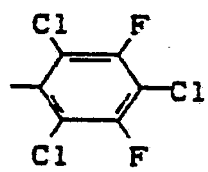 |
| $NO_2$ | H | O | 0 | - | $COOCH_3$ | 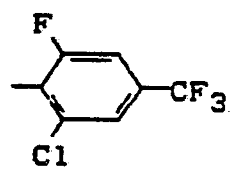 |
| $NO_2$ | H | O | 0 | - | COOH | 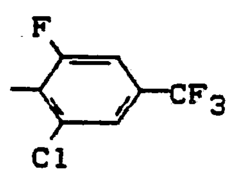 |
| $NO_2$ | H | O | 1 | $CH_2$ | CN | 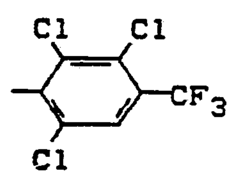 |

Tabelle 1 - Fortsetzung

| $R^1$ | $R^2$ | X | n | A | Y | Ar |
|---|---|---|---|---|---|---|
| $NO_2$ | H | O | 1 | $CH_3$<br>-CH- | CN | 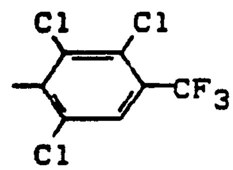 |
| $NO_2$ | H | O | 1 | $-CH_2-$ | CN | 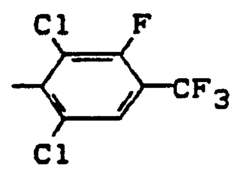 |
| $NO_2$ | H | O | 1 | $CH_3$<br>-CH- | CN | 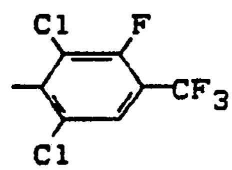 |
| $NO_2$ | H | O | 1 | $-CH_2-$ | CN | 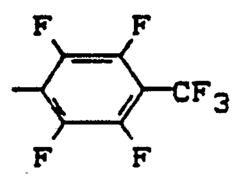 |
| $NO_2$ | H | O | 1 | $CH_3$<br>-CH- | CN | 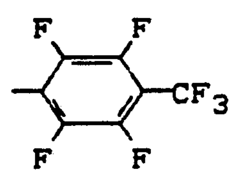 |
| $NO_2$ | H | O | 1 | $-CH_2-$ | CN | 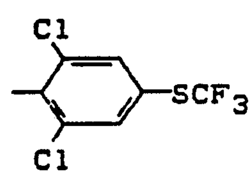 |
| $NO_2$ | H | O | 1 | $-CH_2-$ | CN | 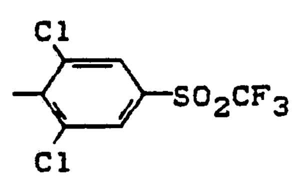 |

Tabelle 1 - Fortsetzung

| $R^1$ | $R^2$ | X | n | A | Y | Ar |
|---|---|---|---|---|---|---|
| $NO_2$ | H | O | 1 | $-CH_2-$ | CN | 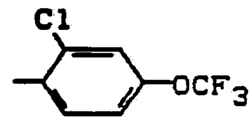 |
| $NO_2$ | H | O | 1 | $-CH_2-$ | CN | 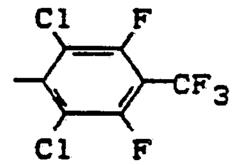 |
| $NO_2$ | H | O | 1 | $-CH_2-$ | $CO_2CH_3$ | 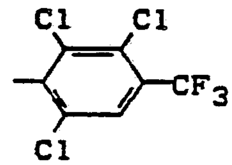 |
| $NO_2$ | H | O | 1 | $CH_3$<br>$-CH-$ | $-CO_2CH_3$ | 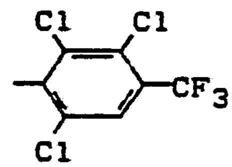 |
| $NO_2$ | H | O | 1 | $CH_3$<br>$-C-$<br>$CH_3$ | $CO_2CH_3$ | 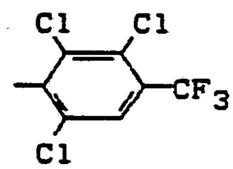 |
| $NO_2$ | H | O | 1 | $-CH_2-$ | COOH | 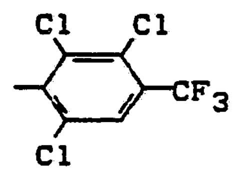 |
| $NO_2$ | H | O | 1 | $CH_3$<br>$-CH-$ | COOH | 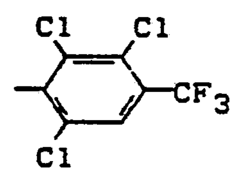 |

Tabelle 1 - Fortsetzung

| $R^1$ | $R^2$ | X | n | A | Y | Ar |
|---|---|---|---|---|---|---|
| $NO_2$ | H | O | 1 | $-CH_2-$ | $CO_2CH_3$ | 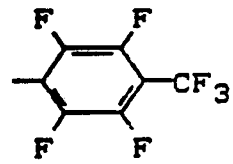 |
| $NO_2$ | H | O | 1 | $-CH_2-$ | $CO_2C_2H_5$ | 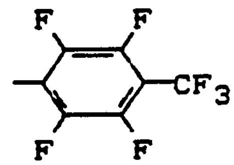 |
| $NO_2$ | H | O | 1 | $-CH_2-$ | COOH | 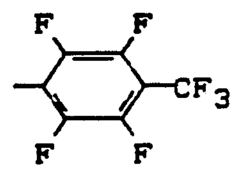 |
| $NO_2$ | H | O | 1 | $CH_3$<br>\|<br>$-CH-$ | $CO_2C_2H_5$ | 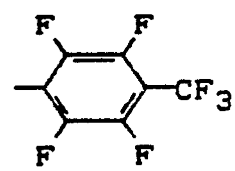 |
| $NO_2$ | H | O | 1 | $-CH_2-$ | $CO_2C_2H_5$ | 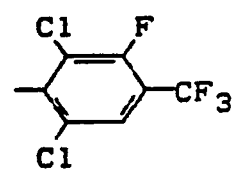 |
| $NO_2$ | H | O | 1 | $CH_3$<br>\|<br>$-CH-$ | $CO_2C_2H_5$ | 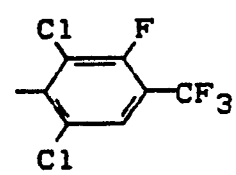 |
| $NO_2$ | H | O | 1 | $-CH_2CH_2-$ | CN | 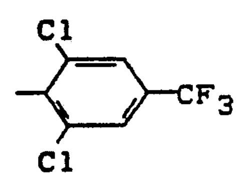 |

Tabelle 1 - Fortsetzung

| $R^1$ | $R^2$ | X | n | A | Y | Ar |
|---|---|---|---|---|---|---|
| $NO_2$ | H | O | 1 | $-CH_2CH_2-$ | $CO_2CH_3$ | 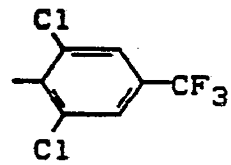 |
| $NO_2$ | H | O | 1 | 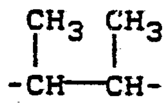 | $CO_2CH_3$ | 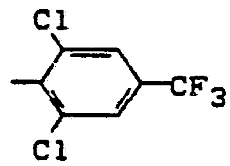 |
| $NO_2$ | H | O | 1 | 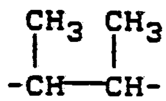 | COOH | 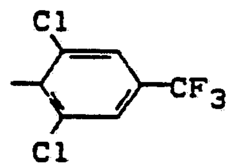 |
| $NO_2$ | H | O | 1 | 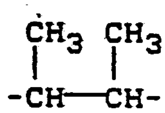 | $COOC_2H_5$ | 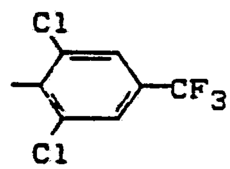 |
| $NO_2$ | H | O | 1 | $CH_2CH_2$ | CN | 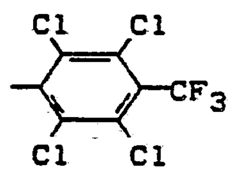 |
| $NO_2$ | H | O | 1 | $CH_2CH_2$ | $COOCH_3$ | 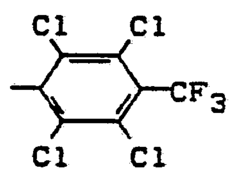 |
| $NO_2$ | H | O | 1 | $-CH_2CH_2-$ | $COOC_2H_5$ | 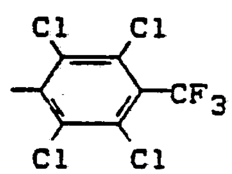 |

<u>Tabelle 1</u> - Fortsetzung

| $R^1$ | $R^2$ | X | n | A | Y | Ar |
|---|---|---|---|---|---|---|
| $NO_2$ | H | O | 1 | $CH_2CH_2$ | COOH | 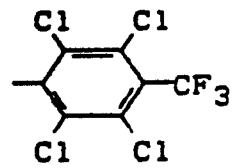 |
| $NO_2$ | H | O | 1 | $-CH_2-$ | CN | 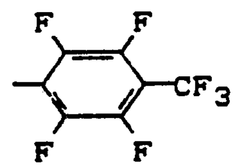 |
| $NO_2$ | H | O | 1 | $-CH_2-$ | $COOCH_3$ | 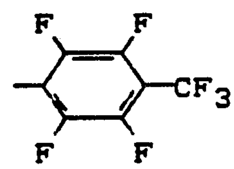 |
| $NO_2$ | H | O | 1 | $-CH_2-$ | $COOC_2H_5$ | 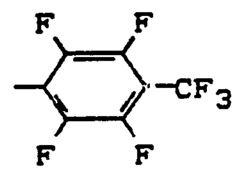 |
| $NO_2$ | H | O | 0 | - | $CONH_2$ | 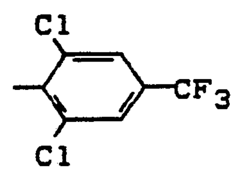 |
| $NO_2$ | H | O | 0 | - | $CON(CH_3)_2$ | 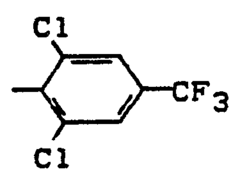 |
| $NO_2$ | H | O | 1 | $-CH_2-$ | $CONH_2$ | 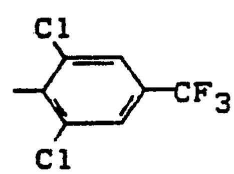 |

<u>Tabelle 1</u> - Fortsetzung

| $R^1$ | $R^2$ | X | n | A | Y | Ar |
|---|---|---|---|---|---|---|
| $NO_2$ | H | O | 1 | $-CH_2$ | $CON(CH_3)_2$ | 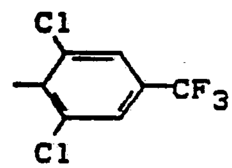 |
| $NO_2$ | H | O | 1 | 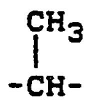 | $CONH_2$ | 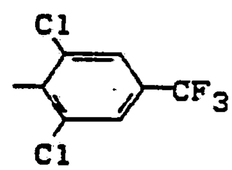 |
| $NO_2$ | H | O | 1 | $-CH_2CH_2-$ | $-CONH_2$ | 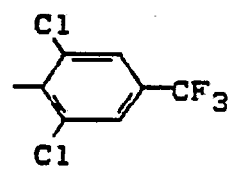 |
| $NO_2$ | H | O | 1 | $-CH_2CH_2-$ | $-CON(CH_3)_2$ | 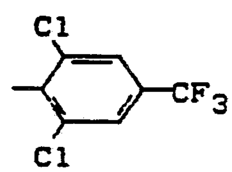 |
| $NO_2$ | H | O | 1 | 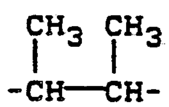 | $-CONH_2$ | 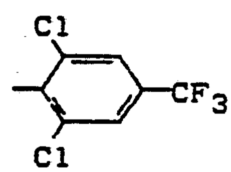 |
| $NO_2$ | H | O | 1 | $-CH_2CH_2-$ | $-CONH_2$ | 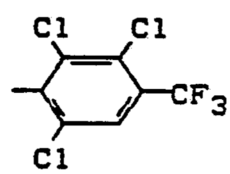 |
| $NO_2$ | H | O | 1 | $-CH_2CH_2-$ | $-CON(CH_3)_2$ | 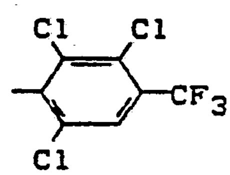 |

<u>Tabelle 1</u> - Fortsetzung

| $R^1$ | $R^2$ | X | n | A | Y | Ar |
|---|---|---|---|---|---|---|
| $NO_2$ | H | O | 1 | $-CH_2-$ | $-CONH_2$ | 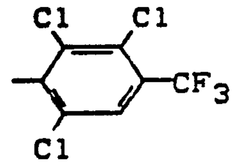 |
| $NO_2$ | H | O | 1 | $-\underset{\mid}{\overset{CH_3}{}}CH-$ | $CONH_2$ | 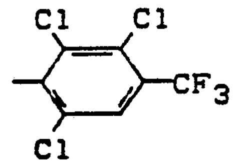 |
| $NO_2$ | H | O | 1 | $-CH_2-$ | $CON(CH_3)_2$ | 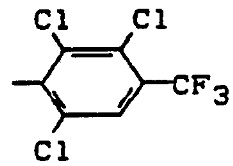 |
| $NO_2$ | $CH_3$ | O | 0 | - | $COOCH_3$ | 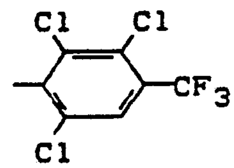 |
| $NO_2$ | $CH_3$ | O | 0 | - | $COOC_2H_5$ | 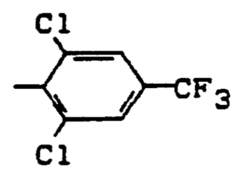 |
| $NO_2$ | $CH_3$ | O | 0 | - | COOH | 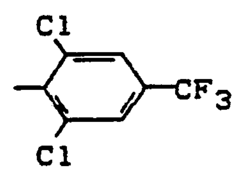 |
| $NO_2$ | $CH_3$ | O | 1 | $-CH_2-$ | $COOC_2H_5$ | 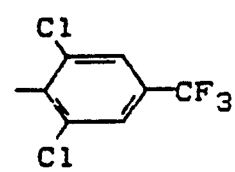 |

Verwendet man beispielsweise 5-Amino-1-(2-brom-4-trifluormethyl-phenyl)-pyrazol und Cyanacetylchlorid als Ausgangsstoffe, so läßt sich der Reaktionsablauf des erfindungsgemäßen Verfahrens (a) durch das folgende Formelschema darstellen:

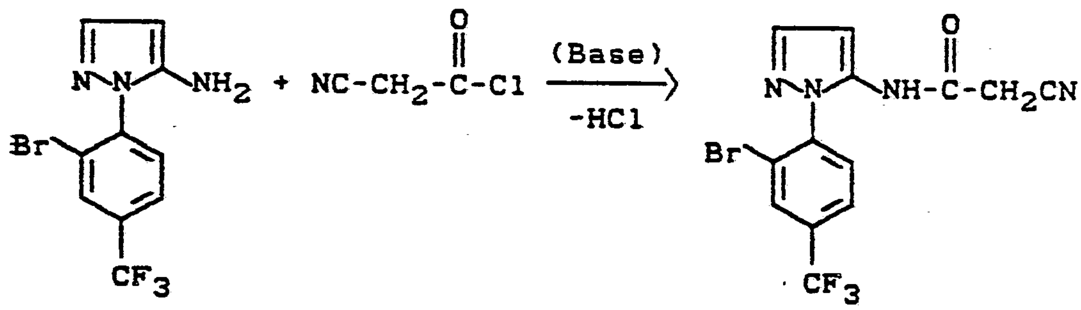


Verwendet man beispielsweise 1-(2-Chlor-4-trifluormethoxyphenyl)-5-ethoxaloyl-amino-4-nitro-pyrazol und Dimethylsulfat als Ausgangsstoffe, so läßt sich der Reaktionsablauf des erfindungsgemäßen Verfahrens (b) durch das folgende Formelschema darstellen:

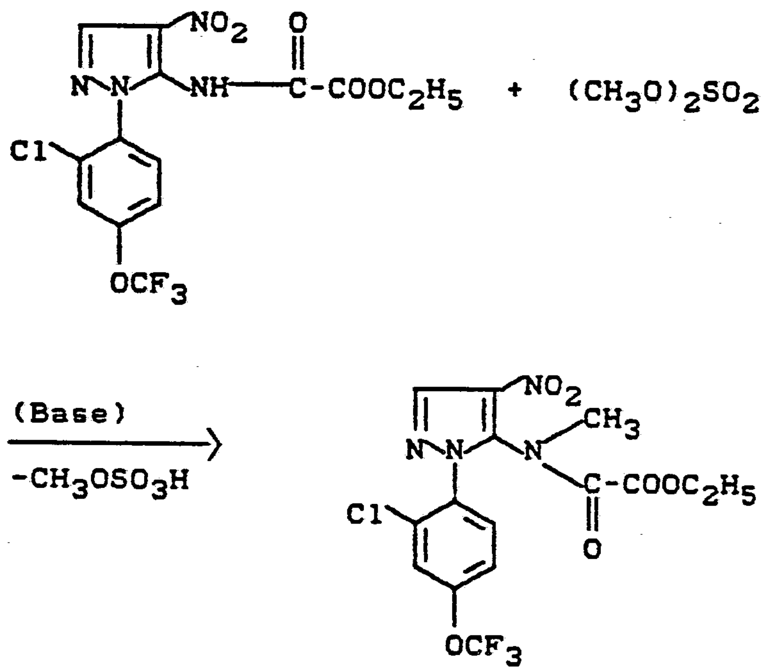


Verwendet man beispielsweise 5-Chloracetylamino-4-nitro-1-(2,4,6-trichlor-phenyl)-pyrazol und Tetrabutyl-ammonium-cyanid als Ausgangsstoffe, so läßt sich der Reaktionsablauf des erfindungsgemäßen Verfahrens (c) durch das folgende Formelschema darstellen:

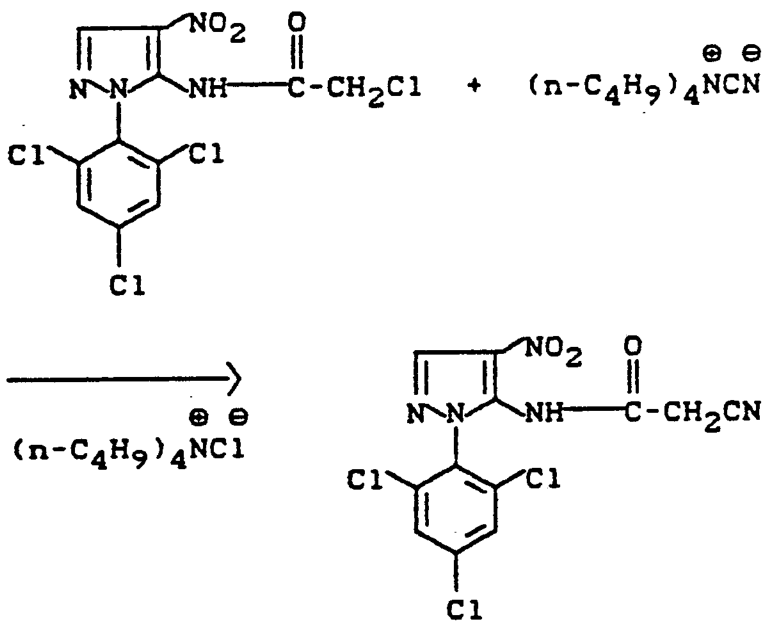


Verwendet man beispielsweise 1-(2,4-Dichlor-phenyl)-5-ethyl-malonoyl-amino-pyrazol und Salpetersäure als Ausgangsstoffe, so läßt sich der Reaktionsablauf des erfindungsgemäßen Verfahrens (d) durch das folgende Formel-schema darstellen:

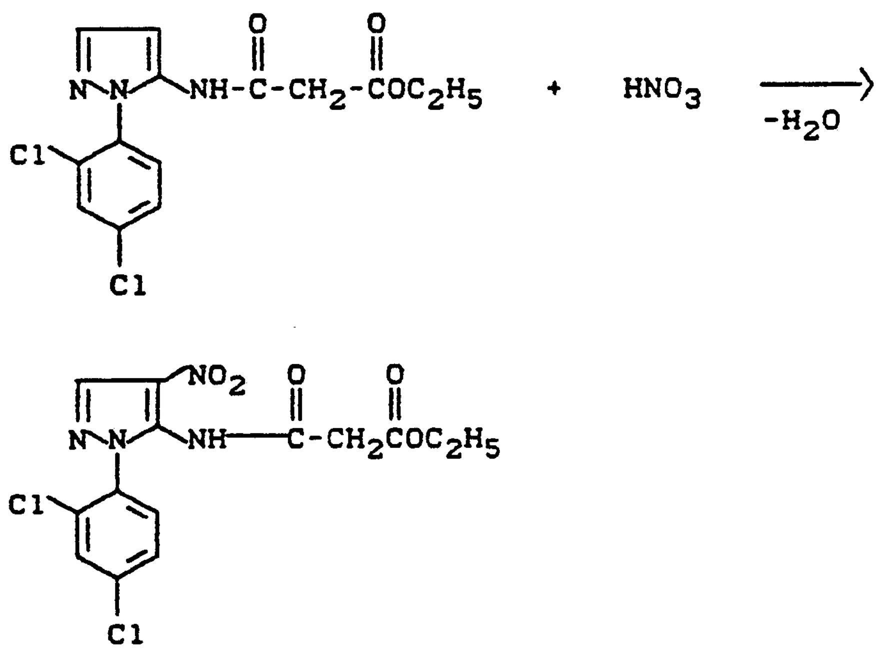


Verwendet man beispielsweise 1-(2,6-Dichlor-4-trifluormethylthio-phenyl)-4-nitro-5-succinimido-pyrazol und Ammoniak als Ausgangsstoffe, so läßt sich der Reaktionsablauf des erfindungsgemäßen Verfahrens (e) durch das folgende Formelschema darstellen:

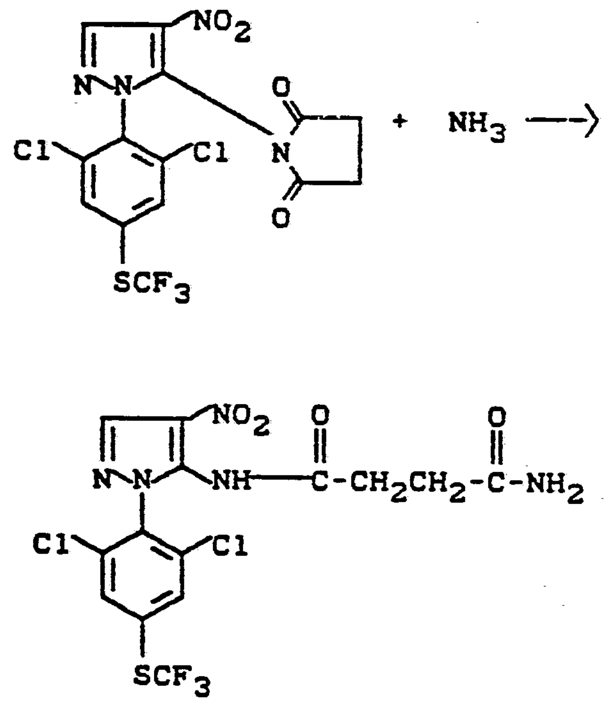


Verwendet man beispielsweise 1-(2,4-Dichlor-phenyl)-5-ethylmalonoyl-amino-pyrazol und Natriumhydroxid als Ausgangsstoffe, so läßt sich der Reaktionsablauf des erfin dungsgemäßen Verfahren (f) durch das folgende Formelschema darstellen:

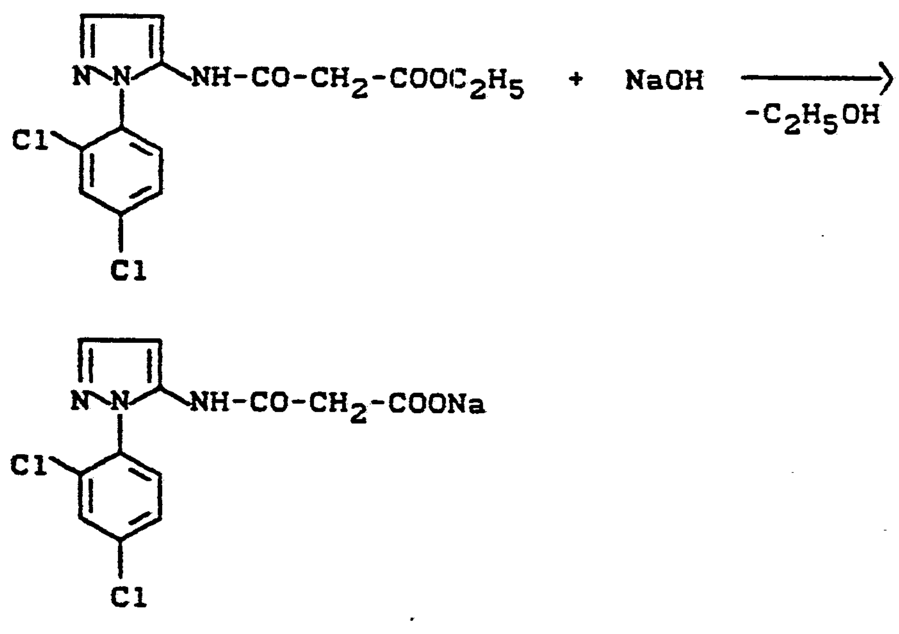


Die zur Durchführung des erfindungsgemäßen Verfahrens (a) als Ausgangsstoffe benötigten 5-Amino-1-aryl-pyrazole sind durch die Formel (II) allgemein definiert. In dieser Formel (II) stehen $R^1$ und Ar vorzugsweise für diejenigen Reste, die bereits im Zusammenhang mit der Beschreibung der erfindungsgemäßen Stoffe der Formel (I) als bevorzugt für diese Reste genannt wurden.

Die 5-Amino-1-aryl-pyrazole der Formel (II) sind teilweise bekannt (vgl. z.B. DE-OS 3 402 308), teilweise sind sie Gegenstand der eigenen vorgängigen noch nicht publizierten Patentanmeldungen DE-Os 3 520 330 vom 07.06.1985 und DE-OS 3 543 033 vom 05.12.1985 und erhältlich in Analogie zu bekannten Verfahren (vgl. DE-OS 3 402 308), beispielsweise wenn man Arylhydrazine der Formel (X),

Ar - NH - $NH_2$ (X)
in welcher
Ar die oben angegebene Bedeutung hat,
mit 2-Halogenacrylnitrilen der Formel (XI),

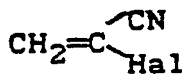

(XI)

in welcher
Hal für Halogen, insbesondere für Chlor oder Brom steht,
entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen -20 °C und +20 °C umsetzt zu den Arylhydrazin-Derivaten der Formel (XII),

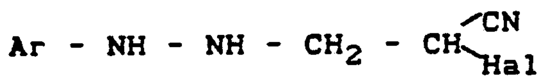

(XII)

in welcher
Ar und Hal die oben angegebene Bedeutung haben,
und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielweise Schwefelsäure oder Phosphorsäure bei Temperaturen zwischen +50 °C und +150 °C cyclisiert,
oder direkt in einem Reaktionsschritt ohne Isolierung der Zwischenstufe der Formel (XII), gegebenenfalls in Gegenwart eines Verdünnungsmittels wie beispielsweise Ethylenglykolmonoethylether oder Ethanol bei Temperaturen zwischen +50 °C und +150 °C cyclisiert und die so erhältlichen 4-unsubstituierten 5-Amino-1-aryl-pyrazole der Formel (IIa),

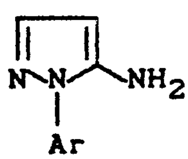

(IIa)

in welcher
Ar die oben angegebene Bedeutung hat,
in einer Folgereaktion mit einem Nitrierungsmittel, wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20 °C und +50 °C nitriert oder alternativ mit einem Halogenierungsmittel, wie beispielsweise Chlor, Sulfurylchlorid, Phosphorpentachlorid, N-Chlorsuccinimid, Brom, Phos phortribromid oder N-Bromsuccinimid, gegebenenfalls in Gegenwart eines Verdünnungsmittels, wie beispielsweise Methylenchlorid oder Eisessig, und gegebenenfalls in Gegenwart eines Reaktionshilfsmittels wie beispielsweise Bortrifluorid bei Temperaturen zwischen -20 °C und +50 °C halogeniert.

Dabei kann es gegebenenfalls von Vorteil sein, vor der Halogenierungs-bzw. Nitrierungsreaktion die Aminogruppe in der 5-Position des Pyrazolringes mit Hilfe üblicher Schutzgruppentechnik beispielsweise durch Acylierung zu schützen und die Aminoschutzgruppe nach erfolgter Halogenierung bzw. Nitrierung ebenfalls in üblicher Art und Weise beispielsweise durch Verseifung mit wässriger oder alkoholischer Base wieder abzuspalten.

Die Arylhydrazine (X) sind bekannt (vgl. z.B. US-PS 4.127.575; US-PS 3.609.158; DE-OS 2 558 399; J. Chem. Soc. C. 1971, 167-174) oder können nach bekannten Verfahren in einfacher analoger Weise erhalten werden (vgl. z.B. Houben-Weyl "Methoden der organischen Chemie" Band X/2, S. 203, Thieme Verlag Stuttgart 1967; DE-OS 3 402 308).

Die Halogenacrylnitrile der Formel (XI) sind allgemein bekannte Verbindungen der organischen Chemie.

Die zur Durchführung des erfindungsgemäßen Verfahrens (a) weiterhin als Ausgangsstoffe benötigten Acylierungsmittel sind durch die Formel (III) allgemein definiert. In dieser Formel (III) stehen X, Y, A und der Index n vorzugsweise für diejenigen Bedeutungen, die bereits im Zusammenhang mit der Beschreibung der erfindungsgemäßen Stoffe der Formel (I) als bevorzugt für diese Substituenten genannt wurden.

$E^1$ steht vorzugsweise für Halogen, insbesondere Chlor oder Brom, oder für einen Rest $Y\text{-}(A)_n\text{-}\underset{\underset{X}{\|}}{C}\text{-O-}$

, wobei X, Y, A und der Index n die oben angegebenen Bedeutungen haben. Die Acylierungsmittel der Formel (III) sind allgemein bekannte Verbindungen der organischen Chemie.

Die zur Durchführung des erfindungsgemäßen Verfahrens (b) als Ausgangsstoffe benötigten 5-Acylamino-pyrazol-Derivate sind durch die Formel (Ia) allgemein definiert. In dieser Formel (Ia) stehen $R^1$, X, Y, A, der Index n und Ar vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5-Acylamino-pyrazol-Derivate der Formel (Ia) sind erfindungsgemäße Verbindungen und erhältlich mit Hilfe der erfindungsgemäßen Verfahren (a), (c), (d) oder (e).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugsweise für geradkettiges oder verzweigtes Alkyl mit 1 bis 12 Kohlenstoffatomen, insbesondere für Methyl, Ethyl, n-oder i-Propyl sowie n-, i-, s-oder t-Butyl; ferner für Alkenyl oder Alkinyl mit jeweils 3 bis 6 Kohlenstoffatomen, insbesondere für Allyl oder Propargyl, und für Cycloalkyl mit 3 bis 6 Kohlenstoffatomen, insbesondere für Cyclopropyl, Cyclopentyl oder Cyclohexyl, $E^2$ steht vorzugsweise für Chlor, Brom oder Iod, für Methoxysulfonyloxy oder für p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten 5-Acylamido-1-aryl-pyrazole sind durch die Formel (V) allgemein definiert. In dieser Formel (V) stehen $R^1$, $R^2$, X, A, Ar und der Index n vorzugsweise für diejenigen Bedeutungen, die bereits im Zuzammenhang mit der Beschreibung der erfindungsgemäßen Stoffe der Formel (I) als bevorzugt für diese Substituenten genannt wurden. $E^3$ steht vorzugsweise für Chlor oder Brom.

Die 5-Acylamido-1-aryl-pyrazole der Formel (V) sind bekannt (vgl. US-PS 4.363.804 oder DE-OS 3 402 308) oder sie sind Gegenstand der eigenen vorgängigen noch nicht veröffentlichten Patentanmeldung DE-OS 3 520 330 vom 07.06.1985 und erhältlich in Analogie zu bekannten Verfahren (vgl. z.B. DE-OS 3 402 308; vgl. auch Herstellungsbeispiele), beispielsweise wenn man 5-Amino-1-aryl-pyrazole der Formel (II),

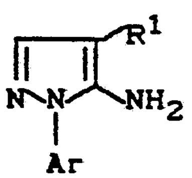

(II)

in welcher

$R^1$ und Ar die oben angegebene Bedeutung haben,

mit Acylierungsmitteln der Formel (XIII),

$E^5\text{-}\underset{\underset{X}{\|}}{C}\text{-}(A)_n\text{-}E^3$

(XIII)

in welcher

$E^3$, X, A und der Index n die oben angegebene Bedeutung haben und

$E^5$ für eine Abgangsgruppe wie beispielsweise Halogen, insbesondere Chlor oder Brom, oder für einen Rest $E^3\text{-}(A)_n\text{-}\underset{\underset{X}{\|}}{C}\text{-O-}$

steht, wobei $E^3$, A, X und der

Index n die oben angegebene Bedeutung haben,

gegebenenfalls in Gegenwart eines Verdünnungsmittels, wie beispielsweise Dichlormethan, und gegebenenfalls in Gegenwart eines Säurebindemittels, wie beispielsweise Pyridin, bei Temperaturen zwischen -20 °C und +120 °C acyliert, und gegebenenfalls anschließend in Analogie zu bekannten Ver fahren und in Analogie zu dem erfindungsgemäßen Verfahren (b) in einer 2. Stufe mit Alkylierungsmitteln der Formel (IV),

$E^2\text{-}R^{2-1}$ (IV)

in welcher
$E^2$ und $R^{2-1}$ die oben angegebene Bedeutung haben,
gegebenenfalls in Gegenwart eines Verdünnungsmittels, wie beispielsweise Acetonitril, und gegebenenfalls in Gegenwart eines Katalysators wie beispielsweise Natriummethylat bei Temperaturen zwischen 0 °C und 80 °C alkyliert.

Die Acylierungsmittel der Formel (XIII) sind allgemein bekannte Verbindungen der organischen Chemie.

Die zur Durchführung des erfindungsgemäßen Verfahrens (c) weiterhin als Ausgangsstoffe benötigten Cyan-Derivate sind durch die Formel (VI) allgemein definiert. In dieser Formel (VI) steht W vorzugsweise für Wasserstoff, für ein Natrium-oder Kaliumkation, oder für ein Tetrabutylammoniumkation.

Die Cyan-Derivate der Formel (VI) sind allgemein bekannte Verbindungen der organischen Chemie.

Die zur Durchführung des erfindungsgemäßen Verfahrens (d) als Ausgangsstoffe benötigten 5-Acylamino-pyrazol-Derivate sind durch die Formel (Ie) allgemein definiert. In dieser Formel (Ie) stehen $R^2$, X, Y, A, Ar und der Index n vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5-Acylamino-pyrazol-Derivate der Formel (Ie) sind erfindungsgemäße Verbindungen und erhältlich mit Hilfe der erfindungsgemäßen Verfahren (a), (b), (c), (e) oder (f).

Die zur Durchführung des erfindungsgemäßen Verfahrens (d) weiterhin als Ausgangsstoffe benötigten Halogenierungs-oder Nitrierungsmittel sind durch die Formel (VII) allgemein definiert. In dieser Formel (VII) steht $R^{1-1}$ vorzugsweise für Chlor, Brom oder Nitro.
$E^4$ steht vorzugsweise für eine übliche Abgangsgruppe, wie beispielsweise Halogen sowie phosphor-ooder -schwefelhaltige halogenierte Abgangsgruppen. Geeignete Halogenierungs-und Nitrierungsmittel sind beispielsweise Salpetersäure, Nitriersäure, Sulfurylchlorid, Phosphoroxychlorid, Phosphoroxybromid, Phosphortribromid und ähnliche allgemein übliche Halogenierungs-und Nitrierungsmittel.

Die Halogenierungs-und Nitrierungsmittel der Formel (VII) sind allgemein bekannte Verbindungen.

Die zur Durchführung des erfindungsgemäßen Verfahrens (e) als Ausgangsstoffe benötigten 5-Imido-pyrazole sind durch die Formel (VIII) allgemein definiert. In dieser Formel (VIII) stehen $R^1$ und Ar vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

$A^1$ steht vorzugsweise für die Alkylenketten

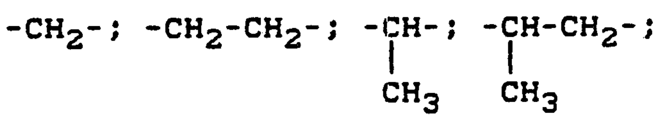

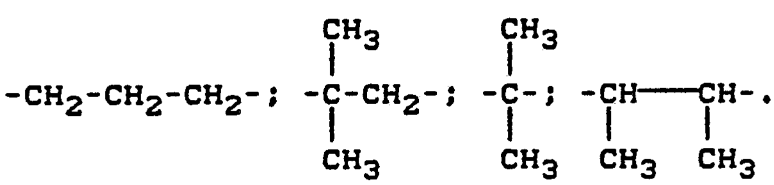

Die 5-Imido-pyrazole der Formel (VIII) sind noch nicht bekannt; sie können jedoch in üblicher Art und Weise erhalten werden, indem man 5-Amino-1-aryl-pyrazole der Formel (II)

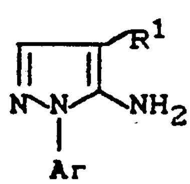 (II)

in welcher
$R^1$ und Ar die oben angegebene Bedeutung haben,
mit cyclischen Dicarbonsäureanhydriden der Formel (XIV)

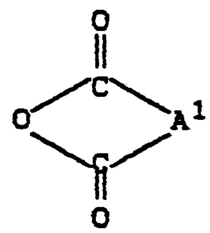

(XIV)

in welcher

$A^1$ die oben angegebene Bedeutung hat,

gegebenenfalls in Gegenwart eines Verdünnungsmittels und gegebenenfalls in Gegenwart eines sauren Katalysators sowie in Gegenwart wasserabspaltender Reagenzien umsetzt, wobei in Abhängigkeit von den Reaktionsbedingungen auch die offenkettigen Amidsäuren der Formel (Ig)

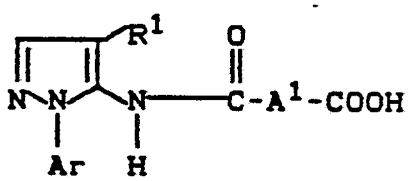

(Ig)

in welcher

$R^1$, $A^1$ und Ar die oben angegebene Bedeutung haben,

isoliert werden können, die dann in einem zweiten Reaktionsschritt unter wasserabspaltenden Bedingungen unter Zusatz eines Reaktionshilfsmittels oder eines Katalysators, wie beispielsweise Dicylohexylcarbodiimid, Trifluoressigsäureanhydrid oder p-Toluolsulfonsäure, zu den 5-Imido-pyrazolen der Formel (VIII) cyclisiert werden.

Als Verdünnungsmittel zur Herstellung der 5-Imido-pyrazole der Formel (VIII) kommen inerte organische Lösungsmittel infrage. Hierbei verwendet man vorzugsweise die beim erfindungsgemäßen Verfahren (a) genannten organischen Solventien. Besonders bevorzugt sind mit Wasser nicht mischbare organische Lösungsmittel, wie Toluol, Chlorbenzol und Tetrachlorkohlenstoff oder organische Säuren, wie Essigsäure und Propionsäure.

Die Herstellung der 5-Imido-pyrazole der Formel (VIII) erfolgt gegebenenfalls unter Verwendung von sauren Katalysatoren, wie Mineralsäuren, Carbonsäuren und Sulfonsäuren, sowie in Gegenwart von wasserabspaltenden Reagenzien, wie Dicyclohexylcarbodiimid oder Säureanhydriden und Säurechloriden, wie Phosporpentoxid, Phosphoroxychlorid, Thionylchlorid umd Trifluoressigsäureanhydrid.

Die Temperaturen können bei dem Verfahren zur Herstellung der 5-Imido-pyrazole der Formel (VIII) in einem größeren Bereich variiert werden. Im allgemeinen arbeitet man bei Temperaturen zwischen 20 °C und 180 °C, vorzugsweise zwischen 50 °C und 180 °C.

Zur Durchführung des Verfahrens zur Herstellung der 5-Imido-pyrazole der Formel (VIII) setzt man pro Mol an 5-Amino-1-aryl-pyrazol der Formel (II) 1 bis 2 Mol, vorzugsweise 1 bis 1,2 Mol an Dicarbonsäureanhydrid der Formel (XIV), 0,01 bis 1 Mol an saurem Katalysator und 1 bis 15 Mol, vorzugsweise 1 bis 10 Mol an wasserabspaltendem Reagenz ein.

Die Reaktionsdurchführung, Aufarbeitung und Isolierung der 5-Imido-pyrazole der Formel (VIII) erfolgt in allgemein üblicher Art und Weise.

Die 5-Imido-pyrazole der Formel (VIII) stellen nicht nur interessante Zwischenprodukte dar, sondern zeigen in entsprechenden Aufwandmengen auch gute herbizide und pflanzenwachstumsregulierende Eigenschaften.

Die zur Durchführung des erfindungsgemäßen Verfahrens (e) weiterhin als Ausgangsstoffe benötigten nucleophilen Verbindungen sind durch die Formel (IX) allgemein definiert. In dieser Formel steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

Die nucleophilen Verbindungen der Formel (IX) sind allgemein bekannte Verbindungen der organischen Chemie.

Die zur Durchführung der erfindungsgemäßen Verfahrens (f) als Ausgangsstoffe benötigten 5-Acylamino-pyrazol-Derivate sind durch die Formel (Ih) allgemein definiert. In dieser Formel (Ih) stehen $R^1$, $R^2$, X, $X^1$, A, Ar und der Index n vorzugsweise für diejenigen Bedeutungen, die bereits im Zusammenhang mit der Beschreibung der erfindungsgemäßen Stoffe der Formel (I) als bevorzugt für diese Substituenten genannt wurden.

$R^{3-1}$ steht vorzugsweise für Hydroxy, Methoxy, Ethoxy, n-und i-Propoxy. Die Verbindungen der Formel (Ih) sind er findungsgemäße Stoffe und erhältlich mit Hilfe der erfindungsgemäßen Verfahren (a), (b), (d) oder (e).

Als Verdünnungsmittel zur Durchführung des erfindungsgemäßen Verfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das erfindungsgemäßen Verfahren (a) kann auch gegebenenfalls in Gegenwart eines geeigneten Acylierungskatalysators durchgeführt werden. Als solche verwendet man vorzugsweise Protonensäure wie Schwefelsäure, Salzsäure, Phosphorsäure, Trifluoressigsäure oder Lewis-Säuren wie Aluminiumtrichlorid, Bortrifluorid oder Eisentrichlorid.

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +150 °C, vorzugsweise bei Temperaturen zwischen 0 °C und 100 °C.

Zur Durchführung des erfindungsgemäßen Verfahrens (a) setzt man pro Mol an 5-Amino-1-aryl-pyrazol der Formel (II) im allgemeinen 1.0 bis 15.0 Mol, vorzugsweise 1.0 bis 5.0 Mol an Acylierungsmittel der Formel (III), gegebenenfalls 1.0 bis 3.0 Mol, vorzugsweise 1.0 bis 2.0 Mol an Säurebindemittel oder gegebenenfalls 0.1 bis 3.0 Mol, vorzugsweise 0,1 bis 2.0 Mol an Acylierungskatalysator ein. Die Reaktionsdurchführung, Aufarbeitung und Isolierung der Reaktionsprodukte der Formel (Ia) erfolgt nach üblichen, bekannten Methoden.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten organischen Lösungsmittel.

Das erfindungsgemäße Verfahren (b) kann gegebenenfalls auch in einem Zweiphasensystem, wie beispielsweise Was ser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens (b) setzt man pro Mol an 5-Acylamino-pyrazol-Derivate der Formel (Ia) im allgemeinen 1.0 bis 20.0 Mol, vorzugsweise 1.0 bis 15.0 Mol an Alkylierungsmittel der Formel (IV) und ge gebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (Ib) erfolgt in allgemein üblicher Art und Weise.

Als Verdünnungsmittel zur Durchführung des erfindungsgemäßen Verfahrens (c) kommen inerte organische Lösungsmittel oder wässrige Systeme infrage. Vorzugsweise verwendet man die bei Verfahren (a) genannten organischen Lösungsmittel. Darüberhinaus bevorzugt sind polare organische Lösungsmittel wie Methanol, Ethanol, n-Propanol, i-Propanol, n-Butanol, i-Butanol, t-Butanol, Ethylenglykol, Diethylenglykol, Ethylenglykolmonomethylether oder -monoethylether, Diethylenglykolmonomethylether oder -monoethylether, deren Gemische mit Wasser oder auch reines Wasser als Verdünnungsmittel.

Das erfindungsgemäße Verfahren (c) wird gegebenenfalls in Gegenwart eines geeigneten Säurebindemittels durchgeführt. Als solche kommen alle üblicherweise verwendbaren anorganischen oder organischen Basen infrage. Vorzugsweise verwendet man die bei Verfahren (b) aufgezählten Basen als Säurebindemittel.

Eine besonders bevorzugte Ausführungsform des Verfahrens (c) besteht darin, daß man Alkali-, Erdalkali-oder Tetraalkylammoniumsalze der Blausäure entweder in inerten organischen Lösungsmitteln oder in einem Zweiphasensystem, wie beispielsweise Wasser/Toluol oder Wasser/Dichlormethan, gegebenenfalls in Gegenwart eines Phasen-Transfer-Katalysators, umsetzt. Vorzugsweise verwendet man die bei Verfahren(b) genannten Katalysatoren.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +120 °C, vorzugsweise bei Temperaturen zwischen 0 °C und +100 °C.

Zur Durchführung des erfindungsgemäßen Verfahrens (c) setzt man pro Mol an 5-Acylamido-1-aryl-pyrazol der Formel (V) im allgemeinen 1.0 bis 5.0 Mol, vorzugsweise 1.0 bis 3.0 Mol an Cyan-Derivat der Formel (VI) und gegebenenfalls 1.0 bis 5.0 Mol, vorzugsweise 1.0 bis 3.0 Mol an Säurebindemittel oder 0.001 bis 2.0 Mol, vorzugsweise 0.01 bis 1.0 Mol an Phasen-Transfer-Katalysator ein. Die Reaktionsdurchführung, Aufarbeitung und Isolierung der Reaktionsprodukte der Formel (Ia) erfolgt in Analogie zu allgemein bekannten Verfahren.

Als Verdünnungsmittel zur Durchführung des Herstellungsverfahrens (d) kommen alle üblicherweise für derartige elektrophile Substitutionen verwendbaren Lösungsmittel infrage. Vorzugsweise verwendet man die als Reagenzien infrage kommenden Säuren oder Gemische, wie beispielsweise Salpetersäure, Nitriersäure, Sulfurylchlorid oder Nitriersäure gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel infrage.

Als Katalysatoren oder Reaktionshilfsmittel zur Durchführung des Herstellungsverfahrens (d) kommen ebenfalls die für derartige Reaktionen üblichen Katalysatoren infrage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (d) in einem größeren Bereich variiert werden. Im allgemeinen arbeitet man zwischen -50 °C und +200 °C, vorzugsweise zwischen -20 °C und +150 °C.

Zur Durchführung des Herstellungsverfahrens (d) setzt man pro Mol 5-Acylamino-pyrazol-Derivat der Formel (Ie) im allgemeinen 1.0 bis 10.0 Mol, vorzugsweise 1.0 bis 5.0 Mol an Halogenierungs-oder Nitrierungsmittel der Formel (VII) und gegebenenfalls 0.1 bis 10 Mol an Katalysator oder Reaktionshilfsmittel ein. Die Reaktionsführung, Aufarbeitung und Isolierung der Reaktionsprodukte der Formel (Id) erfolgt in allgemein üblicher Art und Weise.

Als Verdünnungsmittel zur Durchführung des Herstellungsverfahrens (e) kommen inerte organische Lösungsmittel oder wässrige Systeme infrage. Vorzugsweise verwendet man die bei Verfahren (a) genannten organischen Lösungsmittel. Die Reaktion kann auch in einem Überschuß der nuclephilen Verbindung (IX) als Lösungsmittel durchgeführt werden.

Das erfindungsgemäße Verfahren (e) wird gegebenenfalls in Gegenwart eines Säurebindemittels durchgeführt. Als solche kommen alle üblichen anorganischen oder organischen Basen, wie insbesondere die beim Verfahren (a) bereits genannten, infrage.

Eine bevorzugte Ausführungsform des Verfahrens (e) besteht darin, daß man die nuclophilen Verbindungen der Formel (IX) in Form ihrer Salze einsetzt.

Als Salze kommen hierbei insbesondere die Alkali-, Erdalkali-und Ammoniumsalze der Verbindungen der Formel (IX) infrage.

Die Reaktionstemperaturen können bei der Dürchführung des erfindungsgemäßen Verfahrens (e) in einem größeren Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens (e) setzt man pro Mol 5-Imino-pyrazol der Formel (VIII) im allgemeinen 1 bis 15 Mol, vorzugsweise 1 bis 5 Mol, an nucleophiler Verbindung der Formel (IX), gegebenenfalls 1 bis 3 Mol, vorzugsweise 1 bis 2 Mol, an Säurebindemittel bzw. gegebenenfalls 1 bis 3 Mol, vorzugsweise 1 bis 2 Mol, an Salz der Verbindung der Formel (IX) ein. Die Reaktionsdurchführung, Aufarbeitung und Isolierung der Reaktionsprodukte der Formel (If) erfolgt nach üblichen und bekannten Methoden.

Als Verdünnungsmittel zur Durchführung des erfindungsgemäßen Verfahren (f) kommen inerte organische Lösungsmittel infrage. Vorzugsweise verwendet man die bei Verfahren (a) genannten organischen Lösungsmittel. Bevorzugt können aber auch wässrige oder wässrigorganische Systeme als Verdünnungsmittel verwendet werden.

Als Salzbildner kommen alle üblicherweise verwendeten anorganischen oder organischen Basen infrage. Beispiele für solche Basen sind: Natrium,-Kalium-, Lithium-, Calcium, Barium-und Magnesiumhydroxid-, Natrium-, Kalium-und Calciumcarbonat; Natriumhydrogencarbonat, Kaliumhydrogencarbonat, Natriummethylat, Triethylamin, Isopropylamin sowie Dimethylbenzylamin.

Die Reaktionstemperaturen können bei der Durchführung des erfindungsgemäßen Verfahrens (f) in einem größeren Bereich variiert werden. Im allgemeinen arbeitet man bei Temperaturen zwischen 0 °C und 80 °C, vorzugsweise bei Temperaturen zwischen 20 °C und 50 °C.

Zur Durchführung des erfindungsgemäßen Verfahrens (f) setzt man pro Mol an 5-Acylamino-pyrazol-Derivat der Formel (Ih) im allgemeinen 1 bis 5 Mol, vorzugsweise 1 bis 2 Mol an anorganischer oder organischer Base ein. Die Reaktionsdurchführung, Aufarbeitung und Isolierung der Reaktionsprodukte der Formel (Ig) erfolgt in allgemein üblicher Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkraut-bekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono-und dikotylen Unkräutern in mono-und dikotylen Kulturen, wie beispielsweise Weizen oder Baumwolle einsetzten.

Darüber hinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsions-Konzentrate, wirkstoffimprägnierte Natur-und synthetische Stoffe, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder - schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalo cyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff; N,N-Dimethyl-N'-(4-isopropylphenyl)-harnstoff; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; Methyl-5-(2,4-dichlor phenoxy)-2-nitrobenzoat; 3,5-Diiod-4-hydroxybenzonitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; N-Methyl-2-(1,3-benzthiazol-2-yloxy)-acetanilid; exo-1-Methyl-4-(1-methylethyl)-2(2-methylphenyl-methoxy)-7-oxabicyclo-(2,2,1)-heptan; N,N-Diisoproyl-S-(2,3,3-trichlorallyl)-thiolcarbamat; N-(1-Ethylpropyl)-3,4-dimethyl-2,6-dinitroanilin; 1-Methyl-3-phenyl-5-(3-trifluormethylphenyl)-4-pyridon; 2-[5-Methyl-5-(1-methylethyl)-4-oxo-2-imidazolin-2-yl]-3-chinolincarbonsäure; [(4-Amino-3,5-dichlor-6-fluor-2-pyridinyl)-oxy]-essigsäure; 3,5-Dibrom-4-hydroxy-benzonitril ist möglich.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Bei Anwendung im Nachauflaufverfahren können die erfindungsgemäßen Verbindungen alleine oder in Kombination mit emulgierbaren Ölen, oberflächenaktiven Substanzen und anderen Additiven ausgebracht werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lös liche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

## Herstellungsbeispiele

Beispiel 1

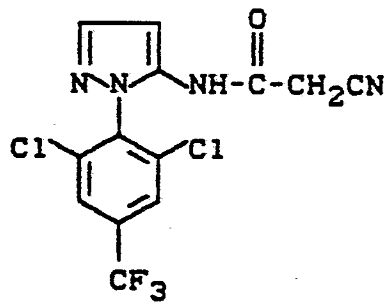

(Verfahren a)

22,5 g (0,076 Mol) 5-Amino-1-(2,6-dichlor-4-trifluormethylphenyl)-pyrazol und 8 g (0,1 Mol) Pyridin werden in 200 ml Acetonitril vorgelegt und unter Rühren und Kühlen bei 20 - 25 °C mit 13,2 g (0,128 Mol) Cyanacetylchlorid tropfenweise versetzt. Es wird noch zwei Stunden bei Raumtemperatur gerührt und der Reaktionsansatz anschließend in Wasser eingegossen. Nach Extraktion mit Methylenchlorid und Trocknen der organischen Phase über Natriumsulfat wird das Lösungsmittel im Vakuum abdestilliert. Der kristalline Rückstand wird mit einem Gemisch aus Ligroin und Diisopropylether verrührt und abgesaugt.

Man erhält 22 g (80% der Theorie) 5-Cyanacetylamino-1-(2,6-dichlor-4-trifluormethyl-phenyl)-pyrazol vom Schmelzpunkt 169 - 175 °C.

Beispiel 2

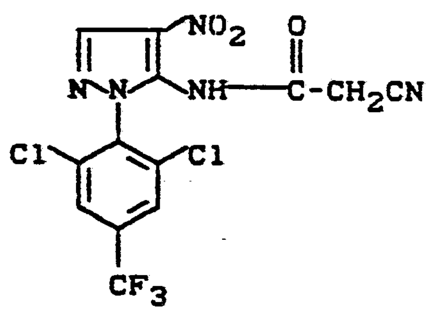

(Verfahren d)

7,26 g (0,02 Mol) 5-Cyanacetylamino-1-(2,6-dichlor-4-trifluormethyl-phenyl)-pyrazol (Bsp. 1) werden in 70 ml Eisessig vorgelegt und nacheinander tropfenweise bei ca. 10 °C mit 2 ml Acetanhydrid und 2 ml Salpetersäure versetzt. Aus der anfänglich klaren Lösung fällt nach längerem Rühren ein kristalliner Feststoff aus. Nach Absaugen, mehrmaligem Waschen mit Diisopropylether und Trocknen erhält man 2,9 g (36 % der Theorie) 5-Cyanacetylamino-1-(2,6-dichlor-4-trifluormethyl-phenyl)-4-nitro-pyrazol vom Schmelzpunkt 221 - 223 °C.

Beispiel 3

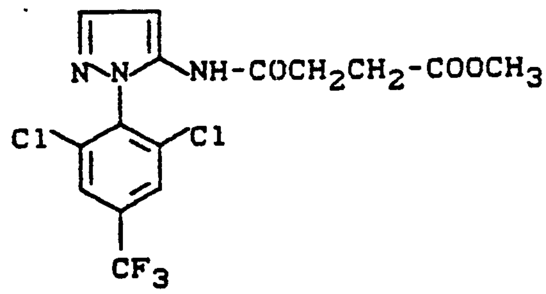

(Verfahren e)

Zu einer Lösung von 8,25 g (0,0218 Mol) 1-(2,6-Dichlor-4-trifluormethyl-phenyl)-5-succinimido-pyrazol in 50 ml wasserfreiem Methanol gibt man bei Raumtemperatur 1,3 g (0,024 Mol) Natriummethylat und rührt 18 Stunden. Danach wird die Reaktionsmischung auf 200 ml 2 %ige Ammoniumchlorid-Lösung und 150 ml Dichlormethan ausgetragen. Die organische Phase wird abgetrennt, mit Wasser und Kochsalzlösung gewaschen, über Magnesiumsulfat getrocknet und im Vakuum vom Lösungsmittel befreit.

Man erhält 7,0 g (78,3 % der Theorie) 1-(2,6-Dichlor-4-trifluormethyl-phenyl)-5-(2-methoxycarbonylpropionylamido)-pyrazol vom Schmelzpunkt 127 - 132 °C.

Herstellung des Ausgangsproduktes

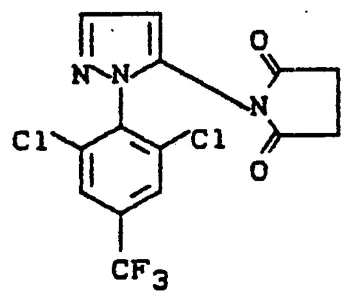

(VIII-1)

Zu einer Lösung von 20 g (0,0676 Mol) 5-Amino-1-(2,6-dichlor-4-trifluormethyl-phenyl)-pyrazol in 150 ml Acetonitril gibt man nacheinander 42 g (0,42 Mol) Bernsteinsäureanhydrid und 40,8 g (0,404 Mol) Triethylamin und erwärmt 16 Stunden auf 80 °C. Das Lösungsmittel wird im Vakuum entfernt und der Rückstand in Wasser aufgenommen. Der entstehende Niederschlag wird abfiltriert, mit Wasser nachgewaschen und getrocknet.

Man erhält 21,6 g (84,5 % der Theorie) 1-(2-6-Dichlor-4-trifluormethyl-phenyl)-5-succinimido-pyrazol vom Schmelzpunkt 162 - 165 °C.

Beispiel 4

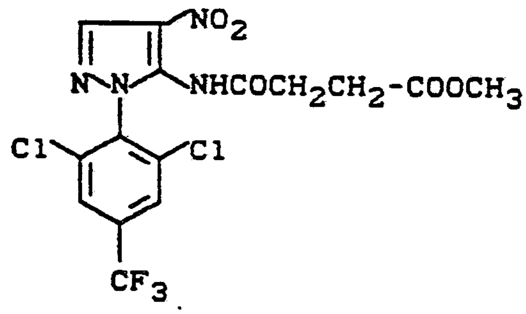

(Verfahren d)

Zu einer Lösung von 4,1 g (0,01 Mol) 1-(2,6-Dichlor-4-trifluormethyl-phenyl)-5-(2-methoxycarbonyl-propionylamido)-pyrazol in 30 ml Eisessig gibt man bei 15 ° - 20 °C nacheinander 1,2 ml (0,013 Mol) Acetanhydrid und 0,5 ml (0,012 Mol) 99 %ige Salpetersäure. Es wird 14 Stunden bei Raumtemperatur gerührt. Danach wird die Reaktonslösung auf 100 ml Wasser ausgetragen. Der Niederschlag wird abfiltriert, mit Wasser neutral gewaschen und im Vakuum getrocknet.

Man erhält 4,1 g (90 % der Theorie) 1-(2,6-Dichlor-4-trifluormethyl-phenyl)-5-(2-methoxycarbonyl-propionylamido)-4-nitro-pyrazol vom Schmelzpunkt 149 - 151 °C.

In entsprechender Weise und gemäß den allgemeinen Angaben zur Herstellung erhält man die folgenden 5-Acylamino-pyrazol-Derivate der allgemeinen Formel (I):

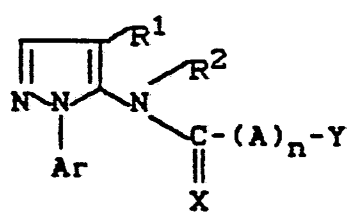

(I)

Tabelle 2

| Bsp. Nr. | $R^1$ | $R^2$ | $-\overset{X}{\overset{\|}{C}}-(A)_n-Y$ | Ar | Fp. (°C) |
|---|---|---|---|---|---|
| 5 | H | H | $-CO-CH_2-CO-OC_2H_5$ | 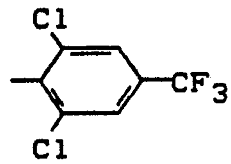 | 104-06 |
| 6 | $NO_2$ | H | $-CO-CH_2-CO-OC_2H_5$ | 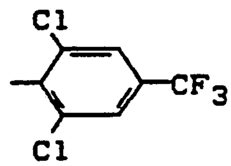 | 107-10 |
| 7 | H | H | $-CO-\overset{CH_3}{\overset{\|}{C}}H-CO-OC_2H_5$ | 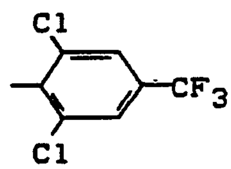 | 115-16 |
| 8 | $NO_2$ | H | $-CO-\overset{CH_3}{\overset{\|}{C}}H-CO-OC_2H_5$ | 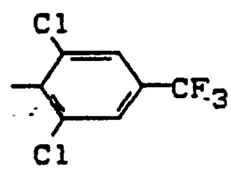 | 120-24 |
| 9 | $NO_2$ | H | -CO-CO-OH | 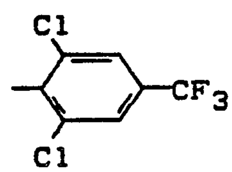 | 211-15 |
| 10 | H | H | $-CO-COOC_2H_5$ | 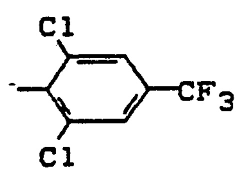 | 119-24 |
| 11 | H | H | $-CO-CO-OC_2H_5$ | 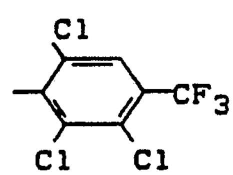 | 109-16 |

<u>Tabelle 2</u> (Fortsetzung)

| Bsp. Nr. | $R^1$ | $R^2$ | $-\overset{X}{\overset{\Vert}{C}}-(A)_n-Y$ | Ar | Fp. (°C) |
|---|---|---|---|---|---|
| 12 | H | H | $-CO-CO-OC_2H_5$ | 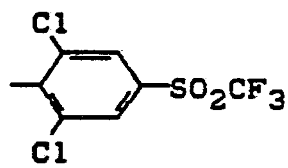 | 103-1 |
| 13 | $NO_2$ | H | $-CO-CO-OC_2H_5$ | 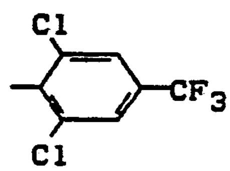 | 127-3 |
| 14 | $NO_2$ | H | -CO-CO-ONa | 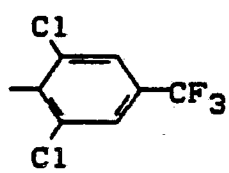 | 228-3 |
| 16 | $NO_2$ | H | $-CO-CH(CH_3)-CO-O-C_2H_5$ | 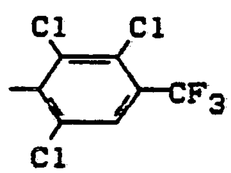 | 108-12 |
| 17 | $NO_2$ | H | -CO-COOH | 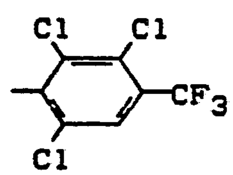 | 178-80 |

Tabelle 2 (Fortsetzung)

| Bsp. Nr. | $R^1$ | $R^2$ | 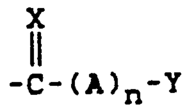 | Ar | Fp. (°C) |
|---|---|---|---|---|---|
| 18 | $NO_2$ | H | $-CO-COOC_2H_5$ | 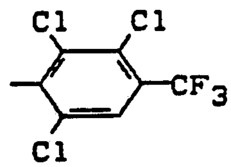 | 117-119 |
| 19 | $NO_2$ | H | $-CO-COOC_2H_5$ | 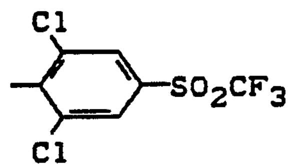 | 138-140 |
| 20 | H | H | $-CO-COOC_2H_5$ | 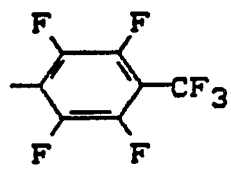 | 85-86 |
| 21 | $NO_2$ | H | $-CO-COONa$ | 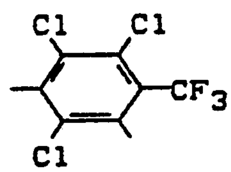 | 175-182 |
| 22 | $NO_2$ | | $-CO-COOC_2H_5$ | 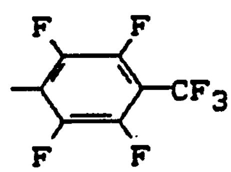 | 136-138 |
| 23 | $NO_2$ | H | $-CO-CH_2-CH_2-COOH$ | 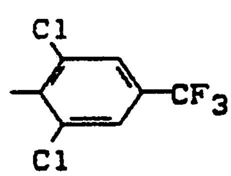 | 176-180 |

Tabelle 2 (Fortsetzung)

| Bsp. Nr. | $R^1$ | $R^2$ | $-\overset{X}{\overset{\|}{C}}-(A)_n-Y$ | Ar | Fp. ($^oC$) |
|---|---|---|---|---|---|
| 24 | H | H | $-CO-COOC_2H_5$ | 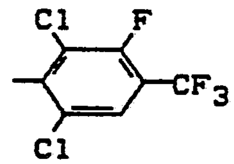 | 104-108 |
| 25 | $NO_2$ | H | $-CO-COOC_2H_5$ | 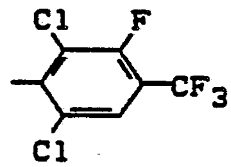 | 124-128 |
| 26 | $NO_2$ | H | $-CO-COOH$ | 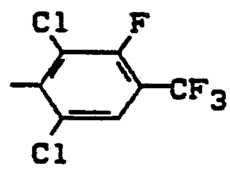 | 193 |
| 27 | $NO_2$ | H | $-CO-CH_2-COOC_2H_5$ | 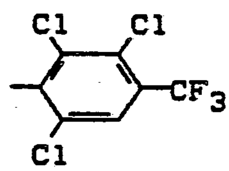 | 108-109 |
| 28 | H | H | $CO-CH_2-CN$ | 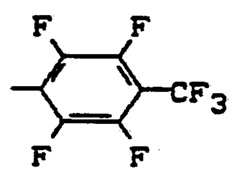 | 142-145 |
| 29 | H | H | $-CO-CH_2-CN$ | 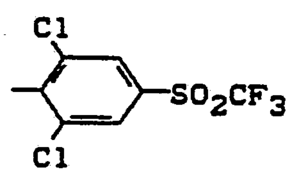 | 155-158 |

<u>Tabelle 2</u> (Fortsetzung)

| Bsp. Nr. | $R^1$ | $R^2$ | $-\overset{X}{\overset{\|}{C}}-(A)_n-Y$ | Ar | Fp. (°C) |
|---|---|---|---|---|---|
| 30 | H | H | $-CO-CH_2-CN$ | 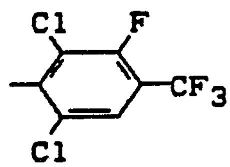 | 174-179 |
| 31 | H | H | $CO-CH_2-CN$ | 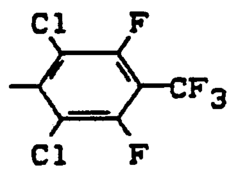 | 194-201 |
| 32 | H | H | $-CO-CH_2-CN$ | 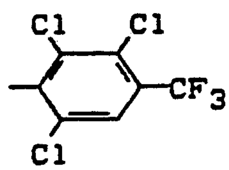 | 147-155 |
| 33 | $NO_2$ | H | $-CO-CH_2-CN$ | 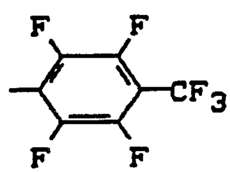 | 148-155 |
| 34 | $NO_2$ | H | $-CO-CH_2-CN$ | 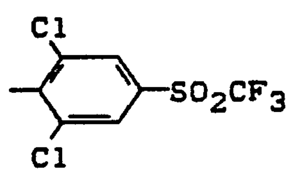 | 183-187 |

<u>Tabelle 2</u> (Fortsetzung)

| Bsp. Nr. | $R^1$ | $R^2$ | X<br>‖<br>$-C-(A)_n-Y$ | Ar | Fp. ($^0$C) |
|---|---|---|---|---|---|
| | $NO_2$ | H | $-CO-CH_2-CN$ | 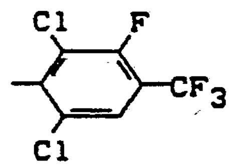 | 180-190 |
| | $NO_2$ | H | $-CO-CH_2-CN$ | 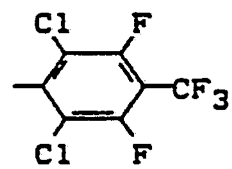 | 180-190 |
| | $NO_2$ | H | $-CO-CH_2-COOCH_3$ | 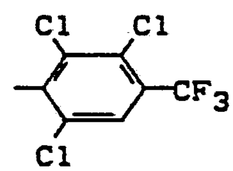 | 163-164 |
| | $NO_2$ | H | $-CO-CH_2-COOCH_3$ | 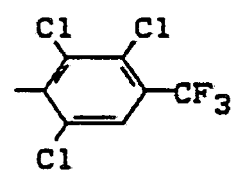 | 163-164 |
| | $NO_2$ | H | $-CO-CH_2-CH_2-COOH$ | 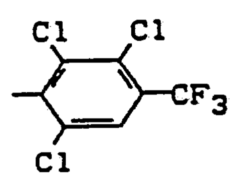 | 204-206 |

Tabelle 2 (Fortsetzung)

| Bsp. Nr. | $R^1$ | $R^2$ | X<br>‖<br>$-C-(A)_n-Y$ | Ar | Fp. ($^0$C) |
|---|---|---|---|---|---|
| | $NO_2$ | H | $-CO-CH_2-CH_2-COOH$ | 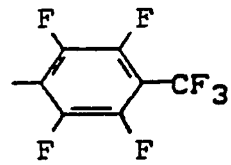 | 146–148 |
| | $NO_2$ | H | $-CO-CH_2-CH_2-COOH$ | 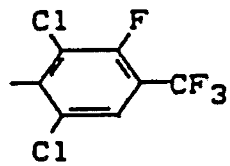 | 175–178 |
| | $NO_2$ | H | $-CO-CH_2-CH_2-COOH$ | 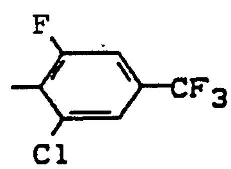 | 177–180 |
| | $NO_2$ | H | $-CO-CH_2-CH_2-COOH$ | 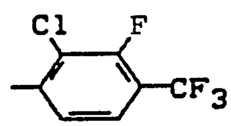 | 112–114 |

Entsprechend Beispiel VIII-1 und gemäß den allgemeinen Angaben zur Herstellung erhält man die folgenden Ausgangsverbindungen der allgemeinen Formel (VIII):

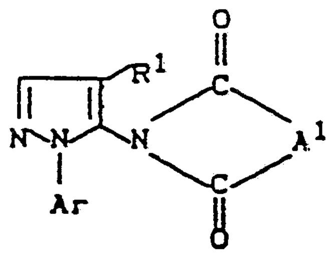

(VIII)

Tabelle 3

| Bsp. Nr. | $R^1$ | $A^1$ | Ar | Fp. (°C) |
|---|---|---|---|---|
| VIII-2 | $NO_2$ | $-CH_2CH_2-$ | 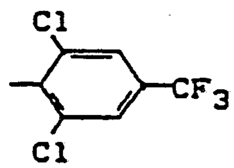 | 152-54 |
| VIII-3 | $NO_2$ | $-CH_2CH_2-$ | 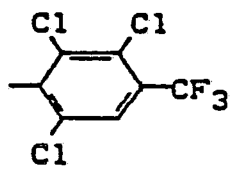 | 174-75 |
| VIII-4 | $NO_2$ | $-CH_2CH_2-$ | 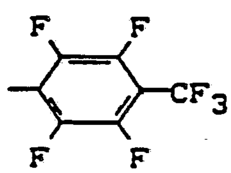 | 163-67 |
| VIII-5 | $NO_2$ | $-CH_2CH_2-$ | 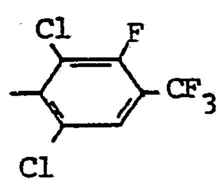 | 128-130 |

Tabelle 3 (Fortsetzung)

| Bsp. Nr. | $R^1$ | $A^1$ | Ar | Fp. (°C) |
|---|---|---|---|---|
| VIII-6 | $NO_2$ | $-CH_2CH_2-$ | 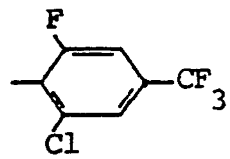 | 138–140 |
| VIII-7 | $NO_2$ | $-CH_2CH_2-$ | 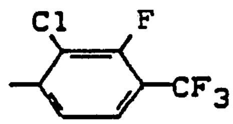 | 168–171 |
| VIII-8 | $NO_2$ | $-CH_2CH_2-$ | 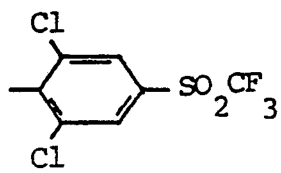 | 242–254 |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z.B. die Verbindungen gemäß den Herstellungsbeispielen: 2, 6, 8, 13 und 14.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z.B. die Verbindungen gemäß den Herstellungsbeispielen: 2, 4, 5, 6, 7, 8, 9, 13 und 14.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

In diesem Test zeigen beispielsweise die Verbindungen gemäß den Herstellungsbeispielen 2, 6, 7, 8 und 14 eine deutliche Überlegenheit im Vergleich zur unbehandelten Kontrolle.

**Ansprüche**

1. 5-Acylamino-pyrazol-Derivate der allgemeinen Formel (I)

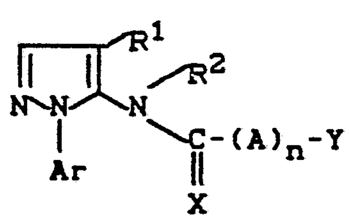

(I)

in welcher
$R^1$ für Wasserstoff, Halogen oder Nitro steht,
$R^2$ für Wasserstoff, Alkyl, Alkenyl, Alkinyl oder gegebenenfalls substituiertes Cycloalkyl steht,
Ar für jeweils gegebenenfalls substituiertes Phenyl oder Pyridyl steht,

X für Sauerstoff oder Schwefel steht,
A für eine geradkettige oder verzweigte, gegebenenfalls substituierte Alkylenbrücke steht,
n für die Zahlen 0 oder 1 steht und
Y für Cyano oder die Gruppierung

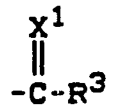

steht, wobei
$X^1$ für Sauerstoff oder Schwefel steht und
$R^3$ für Hydroxy, Alkoxy, Alkenyloxy, Alkinyloxy, Alkylthio, Amino, Alkylamino, Dialkylamino, Alkenylamino, Dialkenylamino, Alkyl-alkenylamino oder einen Rest -OM steht, wobei
M für ein Äquivalent des Kations einer anorganischen oder organischen Base steht.

2. 5-Acylamino-pyrazol-Derivate der Formel (I) gemäß Anspruch 1,
in welcher
$R^1$ für Wasserstoff, Nitro, Fluor, Chlor, Brom oder Iod steht;
$R^2$ für Wasserstoff, für geradkettiges oder verzweigtes Alkyl mit 1 bis 12 Kohlenstoffatomen, für jeweils geradkettiges oder verzweigtes Alkenyl und Alkinyl mit jeweils 3 bis 8 Kohlenstoffatomen sowie für gegebenenfalls durch Alkyl mit 1 bis 4 Kohlenstoffatomen substituiertes Cycloalkyl mit 3 bis 8 Kohlenstoffatomen steht;
Ar für jeweils gegebenenfalls einfach oder mehrfach gleich oder verschieden substituiertes Phenyl, 2-Pyridyl, 3-Pyridyl oder 4-Pyridyl steht, wobei als Substituenten jeweils infrage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$,
wobei
$R^4$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und
m für eine Zahl 0, 1 oder 2 steht; sowie
X für Sauerstoff oder Schwefel steht;
A für eine geradkettige oder verzweigte, gegebenenfalls einfach oder mehrfach, gleich oder verschieden durch Halogen oder Phenyl, das gegebenenfalls durch Halogen oder Alkyl mit 1 bis 4 Kohlenstoffatomen substituiert ist, substituierte Alkylenbrücke mit 1 bis 6 Kohlenstoffatomen steht;
n für eine Zahl 0 oder 1 steht und
Y für Cyano oder die Gruppierung

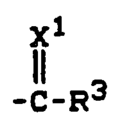

steht,
wobei
$X^1$ für Sauerstoff oder Schwefel steht und
$R^3$ für Hydroxy, jeweils geradkettiges oder verzweigtes Alkoxy und Alkylthio mit jeweils 1 bis 6 Kohlenstoffatomen, jeweils geradkettiges oder verzweigtes Alkenyloxy und Alkinyloxy mit jeweils 3 bis 8 Kohlenstoffatomen, Amino, jeweils geradkettiges oder verzweigtes Alkylamino und Dialkylamino mit jeweils 1 bis 6 Kohlenstoffatomen in den Alkylteilen, jeweils geradkettiges oder verzweigtes Alkenylamino und Dialkenylamino mit jeweils 3 bis 8 Kohlenstoffatomen in den Alkenylteilen, geradkettiges oder verzweigtes Alkyl-alkenylamino mit 1 bis 6 Kohlenstoffatomen im Alkylteil und 3 bis 8 Kohlenstoffatomen im Alkenylteil steht, oder für einen Rest -OM steht,
wobei
M für ein Äquivalent eines Erdalkali-oder Alkalimetallkations oder für ein gegebenenfalls ein-bis vierfach, gleich oder verschieden durch geradkettiges oder verzweigtes Alkyl mit 1 bis 12 Kohlenstoffatomen oder Benzyl substituiertes Ammonium steht.

3. 5-Acylamino-pyrazol-Derivate der Formel (I), gemäß Anspruch 1, in welcher

$R^1$ für Wasserstoff, Nitro, Chlor oder Brom steht;

$R^2$ für Wasserstoff, für geradkettiges oder verzweigtes Alkyl mit 1 bis 8 Kohlenstoffatomen, für jeweils geradkettiges oder verzweigtes Alkenyl und Alkinyl mit jeweils 3 bis 6 Kohlenstoffatomen sowie für gegebenenfalls durch Methyl, Ethyl oder Isopropyl substituiertes Cycloalkyl mit 3 bis 6 Kohlenstoffatomen steht;

Ar für gegebenenfalls ein-bis fünffach, gleich oder verschieden substituiertes Phenyl oder für jeweils gegebenenfalls ein-bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbony, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy Difluorchlormethoxy, Chlormethoxy, Dichlormethoxy, Difluormethoxy, Pentafluorethoxy, Tetrafluor ethoxy, Trifluorchlorethoxy, Trifluorethoxy, Difluordichlorethoxy, Trifluordichlorethoxy, Pentachlorethoxy oder ein Rest $-S(O)_m-R^4$,

wobei

$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und

m für eine Zahl 0, 1 oder 2 steht sowie

X für Sauerstoff oder Schwefel steht;

A für ein Brückenglied der Formel $-CH_2-$; $-CH_2-CH_2-$;

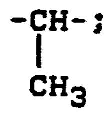 $-CCl_2-CH_2-$; $-CHCl-CH_2-$, 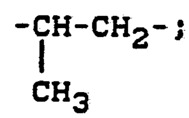

$-CH_2-CH_2-CH_2-$;

$-CH_2-CH_2-CH_2-CH_2-$ ; 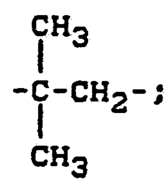 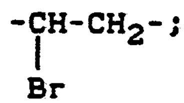 $-CBr_2-CH_2-$;

$-C(CH_3)_2-$; 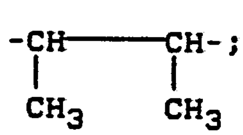 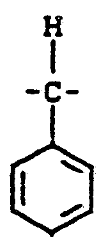 ;

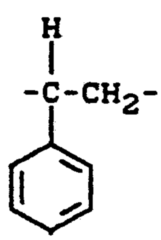 steht;

n für eine Zahl 0 oder 1 steht und

Y für Cyano oder die Gruppierung

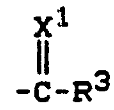

steht,
wobei
$X^1$ für Sauerstoff steht und
$R^3$ für Hydroxy, Alkoxy mit 1 bis 3 Kohlenstoffatomen, Alkenyloxy und Alkinyloxy mit jeweils 3 bis 6 Kohlenstoffatomen, Amino, Alkylamino und Dialkylamino mit jeweils 1 bis 3 Kohlenstoffatomen in jedem Alkylteil, Alkenylamino und Dialkenylamino mit jeweils 3 bis 6 Kohlenstoffatomen in jedem Alkenylteil, Alkylalkenylamino mit 1 bis 3 Kohlenstoffatomen im Alkylteil und 3 bis 6 Kohlenstoffatomen im Alkenylteil steht, oder für einen Rest -OM steht, wobei
M für ein Äquivalent eines Natrium-, Kalium-, Calcium-, Magnesium-oder Barium-Kations oder für ein Isopropylammonium-, Dimethylbenzylammonium-, Triethylammonium-oder Tributylammonium-Ion steht.

4. Verfahren zur Herstellung von 5-Acyl-aminopyrazol-Derivaten der Formel (I)

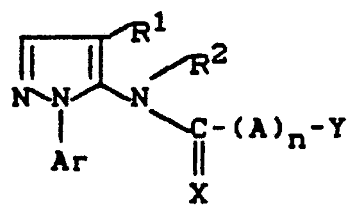
(I)

in welcher
$R^1$ für Wasserstoff, Halogen oder Nitro steht,
$R^2$ für Wasserstoff, Alkyl, Alkenyl, Alkinyl oder gegebenenfalls substituiertes Cycloalkyl steht,
Ar für jeweils gegebenenfalls substituiertes Phenyl oder Pyridyl steht,
X für Sauerstoff oder Schwefel steht,
A für eine geradkettige oder verzweigte, gegebenenfalls substituierte Alkylenbrücke steht,
n für die Zahlen 0 oder 1 steht und
Y für Cyano oder die Gruppierung

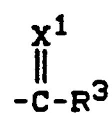

steht, wobei
$X^1$ für Sauerstoff oder Schwefel steht und
$R^3$ für Hydroxy, Alkoxy, Alkenyloxy, Alkinyloxy, Alkylthio, Amino, Alkylamino, Dialkylamino, Alkenylamino, Dialkenylamino, Alkyl-alkenylamino oder einen Rest -OM steht, wobei
M für ein Äquivalent des Kations einer anorganischen oder organischen Base steht,
dadurch gekennzeichnet, daß man
(a) 5-Acylamino-pyrazol-Derivate der Formel (Ia) erhält,

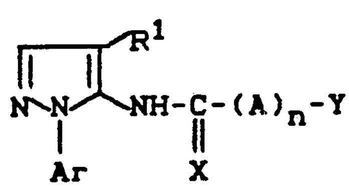
(Ia)

in welcher
$R^1$, X, Y, A, Ar und der Index n die oben angegebene Bedeutung haben,
wenn man 5-Amino-1-aryl-pyrazole der Formel (II)

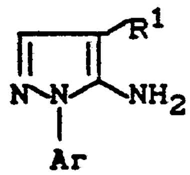

(II)

in welcher
$R^1$und Ar die oben angegebene Bedeutung haben,
mit Acylierungsmitteln der Formel (III)

$$E^1 - \underset{\underset{X}{\|}}{C} -(A)_n -Y$$

(III)

in welcher
X, Y, A und der Index n die oben angegebene Bedeutung haben und
$E^1$ für eine elektronenanziehende Abgangsgruppe steht,
gegebenenfalls in Gegenwart eines Verdünnungsmittels, gegebenenfalls in Gegenwart eines Säurebindemittels sowie gegebenenfalls in Gegenwart eines Katalysators umsetzt;
oder daß man

b) 5-Acylamino-pyrazol-Derivate der Formel (Ib) erhält,

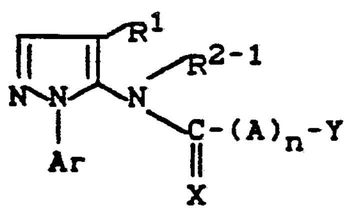

(Ib)

in welcher
$R^1$, X, Y, A, der Index n und Ar die oben angegebene Bedeutung haben und
$R^{2-1}$ für Alkyl, Alkenyl, Alkinyl oder gegebenenfalls substituiertes Cycloalkyl steht,
wenn man die nach Verfahren (a) erhältlichen 5-Acylamino-pyrazol-Derivate der Formel (Ia)

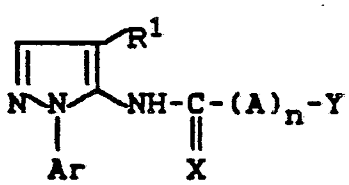

(Ia)

in welcher
$R^1$, X, Y, A, Ar und der Index n die oben angegebene Bedeutung haben,
mit Alkylierungsmitteln der Formel (IV)

$E^2$ -$R^{2-1}$ (IV)

in welcher
$R^{2-1}$ die oben angegebene Bedeutung hat und
$E^2$ für Halogen, für gegebenenfalls substituiertes Alkoxysulfonyloxy oder für gegebenenfalls substituiertes Arylsulfonyloxy steht,
gegebenenfalls in Gegenwart eines Verdünnungsmittels und gegebenenfalls in Gegenwart eines Säurebindemittels sowie gegebenenfalls in Gegenwart eines Katalysators umsetzt;
oder daß man

c) 5-Acylamino-pyrazol-Derivate der Formel (Ic) erhält,

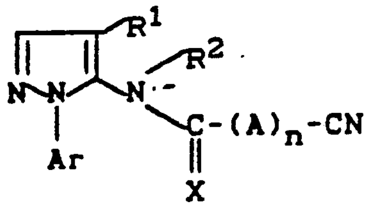

(Ic)

in welcher
$R^1$, $R^2$, Ar, X, A und der Index n die oben angegebene Bedeutung haben,
wenn man 5-Acylamido-1-aryl-pyrazole der Formel (V)

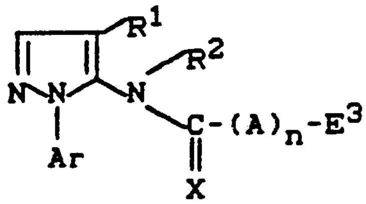

(V)

in welcher
$R^1$, $R^2$, Ar, X, A und der Index n die oben angegebene Bedeutung haben und
$E^3$ für Halogen steht,
mit Cyan-Derivaten der Formel (VI)
W-CN (VI)
in welcher
W für Wasserstoff, ein Äquivalent eines Erdalkali-oder Alkalimetallkations oder für ein Äquivalent des Kations einer organischen Base steht,
gegebenenfalls in Gegenwart eines Verdünnungsmittels und gegebenenfalls in Gegenwart eines Säurebindemittels sowie gegebenenfalls in Gegenwart eines Katalysators umsetzt;
oder daß man

d) 5-Acylaminopyrazol-Derivate der Formel (Id) erhält,

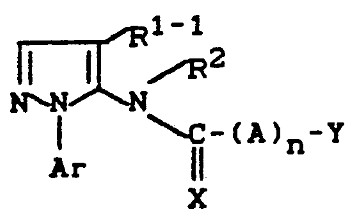

(Id)

in welcher
$R^2$, X, A, Y, Ar und der Index n die oben angegebene Bedeutung haben,
$R^{1-1}$ für Halogen oder Nitro steht,
wenn man die mit Hilfe der Verfahren (a), (b), (c), (e) oder (f) erhältlichen 5-Acylamino-pyrazol-Derivate der Formel (Ie),

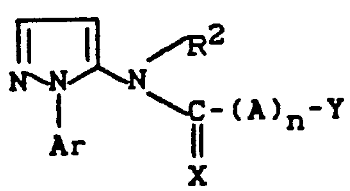

(Ie)

in welcher
$R^2$, X, Y, A, Ar und der Index n die oben angegebene Bedeutung haben,
mit Halogenierungs-oder Nitrierungsmitteln der Formel (VII)
$R^{1-1}$ -$E^4$ (VII)

in welcher
$R^{1-1}$ die oben angegebene Bedeutung hat und
$E^4$ für eine elektronenanziehende Abgangsgurppe steht,
gegebenenfalls in Gegenwart eines Verdünnungsmittels und gegebenenfalls in Gegenwart eines Katalysators oder Reaktionshilfsmittels umsetzt;
oder daß man

e) 5-Acylamino-Pyrazol-Derivate der Formel (If) erhält,

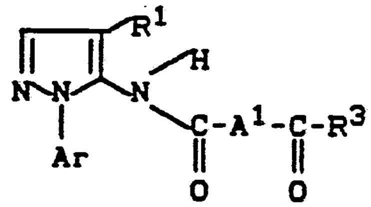
(If)

in welcher
$R^1$, $R^3$ und Ar die oben angegebene Bedeutung haben und
$A^1$ für geradkettiges oder verzweigtes Alkylen mit 1 bis 6 Kohlenstoffatomen steht,
wenn man 5-Imido-pyrazole der Formel (VIII)

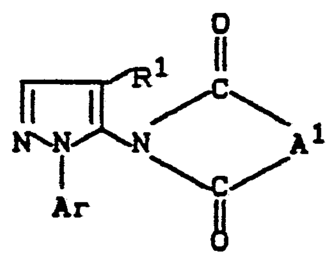
(VIII)

in welcher
$R^1$, Ar, und $A^1$ die oben angegebene Bedeutung haben,
mit nucleophilen Verbindungen der Formel (IX)
$R^3$ -H (IX)
in welcher
$R^3$ die oben angegebene Bedeutung hat,
gegebenenfalls in Gegenwart eines Verdünnungsmittels und gegebenenfalls in Gegenwart eines Reaktionshilfsmittels umsetzt;
oder daß man

f) 5-Acylamino-pyrazol-Derivate der Formel (Ig) erhält,

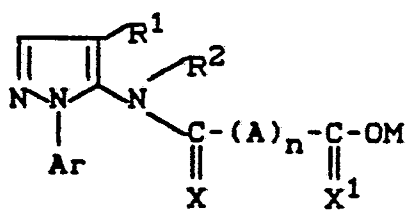
(Ig)

in welcher
$R^1$, $R^2$, Ar, A, X, $X^1$, M und der Index n die oben angegebene Bedeutung haben
wenn man die nach den Verfahren (a), (b), (d) r-oder (e) e hältlichen 5-Acylamino-pyrazol-Derivate der Formel (Ih)

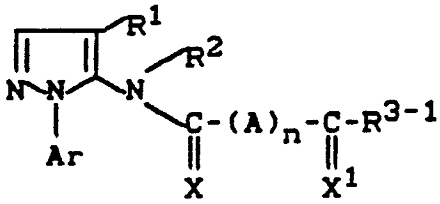

(Ih)

in welcher
$R^1$, $R^2$, Ar, X, $X^1$, A und der Index n die oben angegebene Bedeutung haben und
$R^{3-1}$ für Hydroxy oder Alkoxy steht,
mit den Hydroxiden oder Carbonaten von Alkali-1-oder Erda kalimetallen neutralisiert oder verseift, bzw. mit organischen Basen neutralisiert.

5. Herbizide und pflanzenwuchsregulierende Mittel, gekennzeichnet durch einen Gehalt an mindestens einem 5-Acylamino-pyrazol-Derivate der Formel (I) gemäß den Ansprüchen 1 bis 4.

6. Verfahren zur Bekämpfung von Unkräutern, dadurch gekennzeichnet, daß man 5-Acylamino-pyrazol-Derivate der Formel (I) gemäß den Ansprüchen 1 bis 4 auf die Unkräuter und/oder ihren Lebensraum einwirken läßt

7. Verwendung von 5-Acylamino-pyrazol-Derivaten der Formel (I) gemäß den Ansprüchen 1 bis 4 zur Bekämpfung von Unkräutern und als Pflanzenwuchs-regulatoren.

8. Verfahren zur Herstellung von herbiziden und pflanzenwuchsregulierenden Mitteln, dadurch gekennzeichnet, daß man 5-Acylamino-pyrazol-Derivate der Formel (I) gemäß den Ansprüchen 1 bis 4 mit Streckmitteln und/oder oberflächenaktiven Substanzen vermischt.

9. 5-Imido-pyrazole der Formel (VIII)

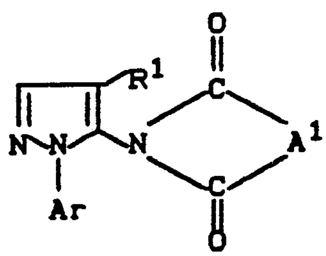

(VIII)

in welcher
$R^1$ für Wasserstoff, Halogen oder Nitro steht,
Ar für jeweils gegebenenfalls substituiertes Phenyl oder Pyridyl steht und
$A^1$ für geradkettiges oder verzweigtes Alkylen mit 1 bis 6 Kohlenstoffatomen steht.

10. Verfahren zur Herstellung von 5-Imido-pyrazolen der Formel (VIII)

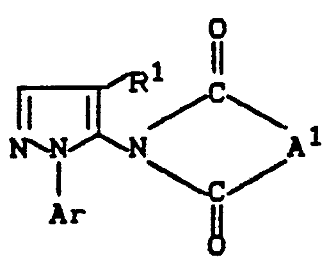

(VIII)

in welcher
$R^1$ für Wasserstoff, Halogen oder Nitro steht,
Ar für jeweils gegebenenfalls substituiertes Phenyl oder Pyridyl steht und
$A^1$ für geradkettiges oder verzweigtes Alkylen mit 1 bis 6 Kohlenstoffatomen,
dadurch gekennzeichnet, daß man
5-Amino-1-aryl-pyrazole der Formel (II)

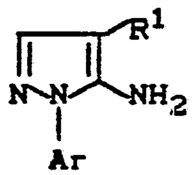

(II)

in welcher
$R^1$ und Ar die oben angegebene Bedeutung haben,
mit cyclischen Dicarbonsäureanhydriden der Formel (XIV)

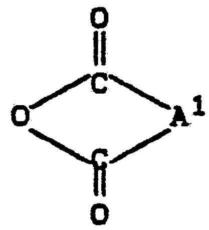

(XIV)

in welcher
$A^1$ die oben angegebene Bedeutung hat,
gegebenenfalls in Gegenwart eines Verdünnungsmittels und gegebenenfalls in Gegenwart eines sauren Katalysators sowie in Gegenwart wasserabspaltender Reagenzien umsetzt.